(19) Europäisches Patentamt
European Patent Office
Office européen des brevets

(11) EP 0 548 334 B1

(12) **FASCICULE DE BREVET EUROPEEN**

(45) Date de publication et mention de la délivrance du brevet: **29.09.1999 Bulletin 1999/39**

(21) Numéro de dépôt: **92915596.8**

(22) Date de dépôt: **03.07.1992**

(51) Int Cl.[6]: **C09K 5/06**, A61F 7/10, F25D 5/00

(86) Numéro de dépôt international: **PCT/FR92/00636**

(87) Numéro de publication internationale: **WO 93/01250 (21.01.1993 Gazette 1993/03)**

# (54) ALLIAGES MOLECULAIRES POUR STOCKER ET RESTITUER L'ENERGIE THERMIQUE PAR CHANGEMENT DE PHASE

Molekulare Legierungen zur Speicherung und Wiedergabe von Wärmeenergie durch Phasenumwandlung

MOLECULAR ALLOYS FOR STORING AND RESTORING THERMAL ENERGY BY PHASE CHANGE

(84) Etats contractants désignés: **AT BE CH DE ES FR GB IT LI LU NL**

(30) Priorité: **10.07.1991 FR 9108695**

(43) Date de publication de la demande: **30.06.1993 Bulletin 1993/26**

(73) Titulaire: **Etablissement Public dit: CENTRE NATIONAL DE LA RECHERCHE SCIENTIFIQUE (CNRS)**
**75007 Paris (FR)**

(72) Inventeurs:
- **HAGET, Yvette**
  **F-33700 Mérignac (FR)**
- **MONDIEIG, Denise**
  **F-33000 Bordeaux (FR)**
- **CUEVAS-DIARTE, Miguel-Angel**
  **E-08006 Barcelona (ES)**

(74) Mandataire: **Peaucelle, Chantal et al**
**Cabinet Armengaud Aine**
**3, avenue Bugeaud**
**75116 Paris (FR)**

(56) Documents cités:

| | |
|---|---|
| **EP-A- 0 022 717** | **EP-A- 0 284 695** |
| **EP-A- 0 344 014** | **EP-A- 0 412 021** |
| **WO-A-85/02009** | **DE-A- 3 141 191** |
| **FR-A- 2 369 529** | |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 548 334 B1

## Description

L'invention a pour objet un procédé d'élaboration de matériaux à changement de phase pour le stockage et la restitution de l'énergie thermique par chaleur latente.
On rappelle qu'il existe deux types principaux de stockage de l'énergie sous forme thermique, à savoir par chaleur sensible et par chaleur latente.
Le stockage par chaleur sensible à travers un matériau procède d'une augmentation de sa température ou, lors de la restitution, d'une diminution de sa température.
Contrairement au stockage par chaleur sensible, le stockage par chaleur latente s'effectue de façon isotherme si le matériau est pur ou avec une certaine variation de température avec les mélanges : c'est le changement de phase du matériau qui est en cause.
Les matériaux à changement de phase ou MCP sont donc des composés susceptibles d'emmagasiner et de restituer de l'énergie thermique par le biais de leurs transitions de phase, le plus souvent de solide à liquide, mais aussi de solide à solide.
Lorsqu'on le chauffe, le matériau prend des calories au milieu extérieur et atteint une température $T_{tr}$, température de transition, passant alors d'une phase 1 à une phase 2 par absorption de chaleur. Lorsque la transition est achevée, sa température peut augmenter à nouveau.
Si on le refroidit, la transition inverse se produit : à $T_{tr}$, le matériau passe de la phase 2 à la phase 1 et restitue au milieu extérieur l'énergie qu'il avait stockée au préalable tout en restant à la température $T_{tr}$. L'énergie mise en jeu est la variation d'enthalpie de changement de phase $\Delta H$.
Des MCP largement utilisés sont constitués par la glace, les hydrates salins. On a également proposé quelques eutectiques. Mais ces matériaux présentent l'inconvénient de fonctionner à une seule température non ajustable. Nombre d'entre eux, en particulier les hydrates salins, sont corrosifs et présentent une fusion incongruente entraînant des phénomènes fréquents de ségrégation difficiles à contrôler, leur conférant une mauvaise tenue au cyclage thermique.
On a également proposé pour le stockage de l'énergie des composés organiques définis, tels qu'acides gras, paraffines, ou encore quelques mélanges de paraffines.
Des mélanges de paraffines solides à 25°C, mis en oeuvre en tant que MCP, sont ainsi décrits dans le brevet FR 2368529. Ce brevet est toutefois axé sur l'amélioration des propriétés thermiques de tels mélanges par addition de métaux, de leurs oxydes ou de leurs silicates. L'exemple donné se rapporte à l'amélioration des propriétés thermiques d'un mélange 82/18 de n-docosane/n-tetracosane par addition d'oxydes de magnésie, d'alumine, d'émeri, de kaolin et d'aluminium à raison de 25 à 55 % en poids.
L'intérêt du mélange de paraffines en tant que tel et les conditions à réunir pour qu'il réponde aux exigences d'une application donnée, à un niveau de température défini, n'y sont toutefois pas envisagés.
Dans les demandes W087/03290 et EP 0344013 et 0344014, on décrit des composites de polyoléfines renfermant des matériaux à changement de phase formés notamment de mélanges de paraffines en C14 ou plus. Ces composites sont prévus pour le stockage de l'énergie solaire.
L'article de Salyer dans 15th North American Thermal Analysis Society Conference Cincinnati, OH, Sep. 21-24, 1986, se rapporte à l'analyse d'hydrocarbures paraffiniques cristallins particulièrement envisagés pour le stockage de l'énergie solaire. Des mélanges de paraffines du commerce sont étudiés au regard de leurs propriétés comme matériaux à changement de phase pour cette application.
Cette étude porte sur des mélanges d'alcanes, selon des proportions données, formés de chaînes impaires, c'est-à-dire à nombre impair d'atomes de carbone (désignées par la suite Cni), (mélanges C15/C19, C17/C19), de chaînes paires, c'est-à-dire à nombre pair d'atomes de carbone (désignées par la suite Cnp) (mélanges C14/C16, C16/C18, C16/C20), de chaînes paires et de chaînes impaires (C16/C17, C18/C19) ou de Cni ou Cnp de chaînes plus longues (> C20). Des mélanges ternaires sont également rapportés (C16/C17/C18 ; C17/C18/C19 ; C16/C18/C20 ; C17/C19/C21 ; C14/C17/C20 et C16/C19/C21), tous pris à la concentration 0,25/0,50/0,25.
De même, Salyer rapporte les résultats obtenus avec des mélanges synthétisés à multicomposants correspondant à des produits du commerce.
Les conditions utilisées pour les mesures ne permettent pas toutefois d'apprécier avec suffisamment de précision les caractéristiques des mélanges étudiés et par là de déterminer pour quel niveau de température ils sont réellement appropriés et ne donnent pas les moyens d'obtenir des compositions parfaitement adaptées à un niveau de température tel que requis précisément dans une application donnée.
Les travaux réalisés par les inventeurs dans ce domaine ont montré que pour disposer de matériaux effectivement appropriés, de grande fiabilité, les composés de départ doivent répondre à des exigences précises, évaluées selon des méthodes rigoureuses.
L'invention a donc pour but de fournir un procédé d'obtention de nouveaux matériaux élaborés au regard de paramètres déterminés, permettant par leur changement de phase de stocker et de restituer des quantités élevées

d'énergie par chaleur latente.

Elle vise en outre l'utilisation de ces matériaux présentant un changement de phase sur un intervalle étroit de température, situé dans un domaine de température permettant de couvrir largement les besoins industriels.

Le procédé d'élaboration de matériaux à changement de phases pour le maintien d'un article au cours de son utilisation à un niveau de température strict, tel que requis pour une application donnée, est caractérisé par la mise en oeuvre d'un ou plusieurs alliages moléculaires, chaque alliage étant formé d'une seule phase constituant, à l'état solide, une solution solide de substitution telle que caractérisée par diffraction X, et répondant strictement à la formule (I) :

$$A_{x_a} Z_{x_z} \qquad (I)$$

dans laquelle

- A et Z sont différents et représentent des composés organiques acycliques ayant de 2 à 120 atomes de carbone, saturés ou insaturés, le cas échéant substitués, présentant la chaleur latente requise de manière classique pour être des matériaux à changement de phase,
  lesdits composés organiques A et Z ayant un degré d'homéomorphisme moléculaire $\varepsilon_K$ supérieur à 0,8 et, de préférence, supérieur à 0,90 pour les alliages binaires, ou ayant cette propriété pour les divers constituants pris deux à deux, s'il s'agit d'alliages à multi-composants,
- $x_a$ et $x_z$ représentent les proportions molaires respectivement de A et de Z, ces proportions étant ajustées pour que les composés, pour chaque alliage moléculaire, soient totalement miscibles avant la transition de phase et que dans le cas de présence de paliers d'invariance, les proportions sont ajustées pour que les composés aient des concentrations situées hors des paliers d'invariance, pour que cette transition s'effectue sur un intervalle de température précis δ n'excédant pas +6°C, incluant la température requise pour une utilisation donnée.

Les matériaux obtenus appartiennent à un diagramme de phase avec, si l'alliage est binaire, un fuseau, dans le cas d'une miscibilité totale, ou une partie de fuseau, dans le cas d'une miscibilité partielle, ou si l'alliage est ternaire ou plus, un domaine de transition, ce fuseau ou ce domaine étant situé dans un intervalle de température incluant celle requise pour une application donnée et dont la courbe EGC (Equal G curve), ou d'une façon générale le lieu géométrique EGC, est peu incurvé et proche de l'horizontalité pour assurer un δ n'excédant pas la largeur souhaitée,

- ont une tenue satisfaisante au cyclage thermique,

ces alliages étant obtenus à partir de composés organiques

. présentent la chaleur latente requise de manière classique pour être des matériaux à changement de phase,

. ont un degré d'homéomorphisme moléculaire $\varepsilon_k$ supérieur à 0,8 et, de préférence, supérieur à 0,90 pour les alliages binaires, ou ayant cette propriété pour les divers constituants pris deux à deux s'il s'agit d'alliages à multicomposants,

. leurs interactions intermoléculaires sont relativement comparables pour les édifices des différents constituants.

Par "alliage moléculaire", on entend une phase unique générée à partir des composés organiques mis en oeuvre pour l'élaborer, cette phase se comportant comme un corps pur du point de vue cristallographique. Cette phase unique, obtenue par syncristallisation, peut être également désignée par les termes de solution solide ou de cristal mixte.

Les analyses par rayons X ont été effectuées par diffractométrie de poudre, et notamment en chambre de Guinier Lenné ou de Guinier Simon.

Par EGC (Equal G Curve), on entend le lieu géométrique des points où solide et liquide (ou solide 1 et solide 2) en équilibre à la transition (pour un binaire), ou d'une façon générale les différentes phases en équilibre à la transition ont la même énergie de Gibbs. (Réf H.A.J. OONK, PHASE THEORY, ELSEVIER 1981).

L'expression "tenue satisfaisante au cyclage thermique" telle qu'utilisée plus haut signifie que des cycles répétés de stockage-destockage dépassant notamment 30 cycles pour les cas d'applications peu répétitives et 5 000 cycles environ pour les cas d'applications très répétitives n'entraînent ni modification chimique, ni ségrégation susceptibles de détériorer le matériau.

Les températures et les enthalpies associées sont mesurées par analyse calorimétrique différentielle, avec étalonnage préalable effectué conformément à l'invention strictement dans les mêmes conditions expérimentales que celles des analyses, ce qui permet de disposer de compositions parfaitement caractérisées notamment du point de vue de leurs propriétés thermodynamiques et qui seront totalement adaptées à l'application envisagée à un niveau donné de température.

Le degré d'homéomorphisme moléculaire $\varepsilon_k$ des composés organiques utilisés pour former l'alliage s'obtient en superposant les deux molécules impliquées de telle sorte que le volume de recouvrement $\Gamma$ soit maximum, leur volume de non recouvrement dans cette position, $\Delta$, est alors minimum. Le degré d'homéomorphisme est donné par la formule :

$$\varepsilon_k = 1 - \frac{\Delta}{\Gamma}$$

Plus les molécules ont des tailles et des formes voisines et plus $\varepsilon_k$ est proche de 1. On trouvera le mode pratique d'obtention de $\varepsilon_k$ dans la référence Haget et al., J. Appl. Cryst (1990), 23, 492-496.

Ainsi, pour des températures variant pratiquement en continu, on dispose de compositions dont le comportement thermique à la fusion ou à la solidification sont comparables à celui des composés purs.

Les alliages moléculaires mis en oeuvre selon l'invention, qui constituent des matériaux à changement de phase et sont désignés également ci-après par l'abréviation MCPAM, présentent une fenêtre d'efficacité thermique $\delta$, définie à 95 % de $\Delta H$.

On rappelle à cet égard que la transition, et en particulier la fusion, d'un alliage est un continuum s'étendant de $T_{solidus}$ à $T_{liquidus}$. L'intervalle de stockage total est égal à $|T_{sol}-T_{liq}|$. La température $T_{95\%}$ (comprise entre $T_{sol}$ et $T_{liq}$) des MCPAM est telle qu'entre $T_{95\%}$ et $T_{liq}$ se trouve stockée 95% de l'énergie de transition.

L'intervalle $\delta = |T_{95\%} - T_{liq}|$ est donc une fenêtre d'efficacité (à 95 %) du MCPAM.

Dans ce qui suit, les MCPAM seront caractérisés par $T\delta$, T étant pris égal à $T_{liq}$.

L'invention vise plus spécialement les matériaux définis ci-dessus dans lesquels $\delta$ n'excède pas +6°C, et ce dans un domaine de température allant de -100°C à +300°C.

Des matériaux particulièrement préférés tels qu'obtenus selon l'invention présentent une fenêtre d'efficacité thermique ne dépassant pas +4°C environ, telle que mesurée dans les conditions strictes évoquées plus haut et avantageusement ne dépassant pas +2°C, voire +1°C.

Dans un mode de réalisation de l'invention, les composés organiques utilisés pour former les alliages moléculaires ne sont pas miscibles en toutes proportions avant la transition, mais le palier d'invariance qui, de ce fait, caractérise le diagramme de phase, est étroit, de l'ordre de quelques pour cents en concentration, et en général déporté vers l'un des composés de départ. Ces formes cristallines impliquées ne sont pas nécessairement isomorphes. Deux cas sont possibles : les formes sont non isomorphes et dans ce cas, il y a 2 types d'alliages solides caractérisés chacun, à une température donnée, par une courbe de G, ou bien les formes des constituants de départ sont isomorphes mais leur degré d'isomorphisme est insuffisant pour conduire à la miscibilité totale ; une seule courbe de G suffit à décrire tous les alliages (à une température T donnée) mais elle présente deux points d'inflexion et seuls sont stables et donc utilisables les alliages dont les concentrations sont extérieures au segment de double tangence.

Dans l'un ou l'autre cas, la (les) partie(s) du diagramme de phase concernée(s) est (sont) celle(s) située (s) hors du palier d'invariance.

Dans un autre mode de réalisation de l'invention, les composés organiques utilisés pour former les alliages moléculaires sont miscibles en toutes proportions avant la transition.

Leurs formes cristallines sont alors isomorphes et leur degré d'isomorphisme cristallin $\varepsilon_m$ proche de 1.

Le degré d'isomorphisme cristallin $\varepsilon_m$ se définit pour deux composés (notion généralisable le cas échéant en prenant les composés deux à deux dans le cas des alliages à multicomposants) en superposant les mailles cristallines des deux composés impliqués de telle sorte que le volume de recouvrement $\Gamma$ soit maximum ; leur volume de non recouvrement dans cette position, $\Delta_m$, est alors minimum et

$$\varepsilon_n = 1 - \frac{\Delta m}{\Gamma m}$$

(Réf. Haget et al donnée ci-dessus).

Le fuseau solidus-liquidus peut prendre trois aspects,

- celui d'un fuseau simple : les alliages que l'on peut fabriquer à partir des composés organiques auront un domaine de fusion intermédiaire entre ceux des constituants de l'alliage,

- celui d'un fuseau à point minimum ; il est alors possible de fabriquer un alliage qui fond à des températures inférieures à celles des composés ;

- celui d'un fuseau à point de Gibbs maximum, certains des alliages obtenus fondant à une température supérieure à celle des composés de départ.

Selon une autre disposition de l'invention, les matériaux en question renferment un ou plusieurs des composés organiques définis ci-dessus à titre d'agents de dopage présents à raison de moins de 5 % en proportions molaires, le ou les composé(s) principal (aux) étant présent(s) à raison de 90 % environ ou plus. Ce dopage permet avantageusement d'ajuster T en fonction d'une application donnée.
Dans les alliages moléculaires mis en oeuvre selon l'invention, on entend, par composé organique acyclique, les composés à chaînes droites ou ramifiées de 2 à 120 atomes de carbone et plus pour les polymères. Ces composés sont de préférence des alcanes, des alcènes ou des alcynes.
Les composés organiques représentés par A et Z comportent le cas échéant un ou plusieurs substituants choisis parmi les halogènes F, Cl, Br, I, les groupes -OH ou -$OR_1$, les groupes alkyle, alkylène et alcyne, ces différents groupes ayant de préférence 1 à 8 atomes de carbone, plus spécialement de 1 à 4 atomes de carbone, les groupes -COOH, -$COOR_1$, -$COR_1$, -$CH_2OH$, -$CH(R_1)$-OH, -$C(R_1, R_2)OH$, ou leurs éthers,

$$-CHO, \quad -\overset{|}{C} = O \quad -CONH_2,$$

-$NH_2$, $NH(R_1)$, ou -N ($R_1$, $R_2$), = S, -SH, -$NO_2$, $R_1$ et $R_2$, identiques ou différents l'un de l'autre, étant un groupe alkyle de 1 à 8 atomes de carbone, de préférence de 1 à 4 atomes de carbone, des groupes fonctionnels tels que amine, cétone, sulfhydryle, amide, pouvant constituer un ou plusieurs maillons de la chaîne. Les substituants des composés organiques acycliques comprennent également les cycles et hétérocycles énumérés plus haut.
Dans les composés organiques ou leurs éthers acycliques, les substituants ci-dessus occupent une position quelconque sur la chaîne carbonée, et/ou se trouvent à une extrémité de la chaîne ou à chacune des deux extrémités.
Dans une famille d'alliages moléculaires utilisés selon l'invention, A et Z appartiennent à la même classe de composés.
Un groupe de cette famille est formé de chaînes hydrocarbonées, plus spécialement d'alcanes, d'alcènes ou d'alcynes, à l'exclusion des mélanges expressément ponctuels décrits dans les documents de l'art antérieur cités plus haut.
Des alliages moléculaires particulièrement préférés sont élaborés à partir d'alcanes renfermant de 8 à 100 atomes de carbone, à chaînes droites ou ramifiées, le cas échéant substitués comme indiqué ci-dessus.
Les alliages moléculaires élaborés à partir d'alcanes normaux, de formule chimique $C_nH_{2n+2}$, ou en abrégé $C_n$, seront désignés ci-après par le terme ALCAL.
Dans cette famille, toutes les interactions intermoléculaires sont de type Van Der Waals, donnant des $_{\Delta}G_{excès}$ très faibles aussi bien pour les solutions solides que pour les solutions liquides ce qui induit des courbes EGC peu incurvées.
Les ALCAL dits recevables, c'est-à-dire répondant aux exigences définies plus haut, présentent une excellente tenue au cyclage thermique ; ils peuvent subir plusieurs milliers de cycles sans dommage (à condition d'éviter la sublimation qui pourrait fausser leur teneur). Ceci est dû aux actions conjointes du $\delta$ faible, du fait qu'ils sont chimiquement inertes et que leurs densités sont voisines. De plus les phénomènes de surfusion sont très faibles voire inexistants, si l'on s'en tient à des conditions d'utilisation proches de celles des applications envisagées plus loin.
Ils présentent en outre l'avantage d'être chimiquement inertes, non corrosifs, imperméables à l'eau et recevables du point de vue des exigences sanitaires.
Des ALCAL recevables, couvrant un domaine de température correspondant à une large gamme telle que requise le plus généralement dans l'industrie, sont formés de chaînes allant de C8 à C100, plus spécialement de C8 à C50.
Dans ces ALCAL, les chaînes constitutives sont Cnp ou Cni ou encore Cnp et Cni. Ces chaînes peuvent être consécutives du type Cni/Cni, ou Cnp/Cnp ou Cni/Cnp, ou Cnp/Cni. En variante, elles diffèrent de plusieurs chaînons, notamment de plus de 4 ou 5 chaînons carbonés. On citera par exemple des ALCAL renfermant au moins une chaîne C14 ou plus, la ou les autres chaînes comportant plus de 5 chaînons carbonés supplémentaires.
Selon une disposition particulière de l'invention, les ALCAL ci-dessus comportent au moins une chaîne avec un ou plusieurs substituants tels que définis ci-dessus.
Parmi ces substituants, on citera les groupes carboxyle, esters et halogènes, qui peuvent occuper avantageusement l'une ou chacune des extrémités de la chaîne.

Selon les exigences à satisfaire pour une application donnée, l'homme de l'art choisira les substitutions les plus appropriées pour obtenir la valeur T souhaitée. De même les proportions de chaque chaîne seront aisément choisies en considérant les diagrammes de labels δ comme illustré par les exemples.
Comme indiqué plus haut, un ou plusieurs des constituants de l'ALCAL peut être présent à titre d'agent de dopage. La proportion molaire de chaque dopant est alors de l'ordre de moins de 5 %.
On rappelle que, sous une forme avantageuse de l'invention, ces différents alliages sont dopés.
Dans encore un autre groupe mis en oeuvre dans l'invention, les composés organiques sont des chaînes polymères.
Dans une autre famille d'alliages utilisés selon l'invention, A et Z appartiennent à des classes différentes de composés.
Des alliages préférés de ces différentes familles sont binaires et répondent à la formule (II) :

$$A_{xa}\ B_{xb} \qquad (II)$$

avec $x_a + x_b = 1$, soit encore $A_{1-x}\ B_x$
D'autres alliages sont ternaires et présentent la formule (III) :

$$A_{xa}\ B_{xb}\ C_{xc} \qquad (III)$$

avec $x_a + x_b + x_c = 1$
D'autres alliages encore sont quaternaires et répondent à la formule (IV) :

$$A_{xa}\ B_{xb}\ C_{xc}\ D_{xd} \qquad (IV)$$

avec $x_a + x_b + x_c + x_d = 1$
Dans ces formules les divers constituants A, B et le cas échéant C et D et les indices correspondant aux concentrations molaires respectives, répondent aux exigences définies ci-dessus et B, C et D présentent les significations données plus haut pour A, mais sont différents les uns des autres.
D'autres alliages renferment plus de quatre composés organiques, à savoir 5, 6 ou plus.
Pour la préparation de ces matériaux on met en oeuvre les composés organiques définis ci-dessus selon les proportions souhaitées dans l'alliage final et on les soumet à des techniques classiques telles que la fusion-cristallisation, la fusion-trempe, la dissolution-évaporation, la sublimation simultanée, la zone levelling ou l'interdiffusion chimique. La technique de zone levelling est décrite notamment par Kolkert W.J, thèse 1974, Utrecht, Pays-Bas.
Dans un mode de réalisation avantageux, pour préparer les ALCAL, lorsque tous les Cn sont liquides à température ambiante, on forme la solution liquide recherchée par mélange et agitation des composés de départ utilisés selon les proportions souhaitées dans l'alliage. Lorsque tous les Cn de départ, ou certains d'entre eux sont solides, on procède par dissolution des produits de base selon les proportions appropriées dans un solvant commun tel que l'éther, suivie par l'évaporation du solvant (de préférence sous courant de gaz inerte). En variante, les produits pesés sont soumis à une fusion en agitant pour assurer l'homogénéité du produit, puis sont trempés.
En variante, on opère selon des méthodes séparatives, ce qui présente l'intérêt d'utiliser les sous-produits de l'industrie pétrolière. Ainsi, on soumet un mélange de composés organiques renfermant la composition recherchée à une étape d'extraction afin d'isoler cette dernière. Lorsque ce mélange ne renferme pas l'ensemble des composés organiques souhaités et/ou selon les proportions recherchées, on traite le mélange de manière à l'enrichir en un ou plusieurs constituants ou, selon le cas, de manière à les éliminer, jusqu'à l'obtention de la composition recherchée.
De manière générale l'invention fournit, pour un même niveau de température, plusieurs formulations recevables, ce qui permet un choix selon les conditions économiques et/ou la disponibilité des produits de base.
La mise en oeuvre d'alliages offre en effet la possibilité de choisir la teneur et donc de rechercher les formulations adéquates pour un rendement optimum de l'énergie stockée et/ou pour satisfaire aux exigences quant à la température d'utilisation et/ou quant à la fenêtre thermique δ souhaitée.
Les travaux réalisés ont permis de montrer la faisabilité d'au moins trois fonctions de grand intérêt, à savoir celles de

- capteur d'énergie : les alliages moléculaires permettent de stocker et de restituer d'importantes quantités de chaleur et ceci pratiquement à température constante ; à l'échelle du kg, et même du mg pour le cas particulier des

ALCAL, la surfusion est très faible voire inexistante.

- lissage de température : un espace entouré par un alliage moléculaire de l'invention peut être maintenu approximativement à la température T (comprise dans l'intervalle de transition de l'alliage). Lorsque l'extérieur subit de larges fluctuations thermiques, l'accroissement en température est réduit par le stockage de chaleur sous forme de chaleur latente, et tout abaissement brusque en température sera lui-même minimisé par la transformation inverse,

- écran thermique : les alliages moléculaires peuvent être utilisés pour ralentir l'effet d'une onde thermique.

Les tests effectués (échelles mg, g, kg) sur les alliages moléculaires de l'invention ont montré que ces matériaux possèdent notamment d'excellents rendements et fiabilités thermiques et que leur tenue au cyclage thermique est remarquable.

A titre d'exemple, une garantie de tenue de 30 ans de service peut être assurée dans un cyclage au rythme journalier pour les alliages moléculaires dont le domaine de fusion n'excède pas 4°C et dont la densité des constituants est proche, ce qui est le cas des alcanes. (Toutes propriétés que ne possèdent pas les hydrates salins, MCP classiques).

Les propriétés avantageuses des matériaux définis ci-dessus sont mises à profit en les utilisant en tant que matériaux à changement de phase pour le stockage et la restitution de l'énergie à un niveau de température donné tel que requis dans une application particulière.

L'invention vise donc l'application des matériaux à changement de phase, élaborés à partir d'un ou plusieurs alliages moléculaires, comme défini ci-dessus, pour stocker et restituer l'énergie thermique à un niveau de température T, sur un intervalle de température δ, correspondant strictement à ceux requis pour une application donnée.

L'invention vise en particulier en tant que MCPAM, l'utilisation des différents alliages $A_{xa}\ Z_{xz}$ définis plus haut dans des conditionnements appropriés pour une application donnée.

Dans ces applications, le principe de base repose sur le fait qu'une situation de non équilibre thermodynamique va être créée et va nécessairement être suivie d'un chemin allant vers le retour à l'équilibre . Le MCPAM va soit se réchauffer en prenant des calories au milieu extérieur pour fondre, soit se refroidir en restituant des calories au milieu extérieur pour se solidifier. Dans les deux cas, la transition se manifestera par un long palier quasi isotherme, (sa pente sera d'autant plus faible que δ sera plus petit).

Grâce aux caractéristiques évoquées ci-dessus, les MCPAM tels qu'obtenus selon l'invention permettent de répondre à de nombreux besoins non satisfaits à ce jour, dans des domaines très variés. On citera par exemple les domaines agro-alimentaire et paramédical, et ceux liés à la protection ou à des utilisations domestiques.

Ainsi, dans le domaine agro-alimentaire, tous les problèmes de non rupture de la chaîne de froid sont concernés. Lesdits alliages peuvent fournir des réponses pour la protection thermique et/ou le transport de denrées alimentaires typiquement de -50°C à +100°C environ.

Dans la gamme de -50°C à -10°C environ, ces MCPAM sont particulièrement appropriés pour le transport en gros, ou à l'échelle individuelle des produits congelés, ou pour leur conservation. Leur utilisation permet ainsi de se prémunir contre des coupures de courant dans les congélateurs, (grandes surfaces et familial) en équipant ces derniers des MCPAM appropriés.

L'utilisation selon l'invention des MCPAM décrits plus haut revêt également un grand intérêt dans le domaine de température de -10°C à +6°C environ. Ainsi, elle permet d'assurer dans les meilleures conditions le transport de boissons et de glaces par exemple dans des dispositifs de type glacière tout en les conservant à la température souhaitée. Il est également possible de réaliser des assiettes, plats ou porte-plats assurant la fraîcheur requise.

Les MCPAM recevables dans ce domaine de température sont également utilisables pour élaborer des étals réfrigérés, ce qui répond par exemple aux besoins des poissonniers.

Les MCPAM s'avèrent en outre précieux dans une gamme de température entre +6°C et +16°C qui est celle requise pour les denrées alimentaires ne devant pas subir de congélation mais nécessitant une conservation et/ou une dégustation à une température relativement basse, par exemple les produits laitiers, les produits de quatrième gamme et les vins chambrés.

Les MCPAM ayant une température de transition de phase supérieure à +16°C, et notamment jusqu'à +35°C présentent un intérêt pour chambrer certains autres vins nécessitant une température supérieure pour leur dégustation, et en cuisine pour des beurriers, des saucières, des yaourtières ou des conditionnements pour des pâtes à levain, ou pour le maintien de pâtisseries ou tartes tièdes.

Dans des domaines de températures plus élevées, de +35°C à +100°C environ, les MCPAM définis plus haut sont utiles pour assurer une stabilisation thermique de dispositifs, par exemple de fermenteurs de +35°C à +37°C, ou pour le maintien au chaud, à température stable, jusqu'à des températures atteignant +100°C environ. On citera par exemple le maintien au chaud de plats cuisinés (ce qui revêt un grand intérêt pour les traiteurs, en particulier lors de

livraisons à domicile, les cafétérias, les restaurants d'entreprise, les grandes surfaces), plats, porte-plats, biberons, assiettes à bouillie, assiettes chaudes, chauffe-plats, plateaux repas, boissons chaudes.

Un marqueur de couleur et/ou de flaveur de qualité alimentaire sera associé avantageusement à l'ALCAL pour alerter le consommateur en cas d'écoulement accidentel dans l'aliment.

Des MCPAM particulièrement appropriés pour ces applications sont formés d'alliages moléculaires ayant une fenêtre thermique ne dépassant pas +2°C, ou mieux +1°C, ou encore moins.

Les ALCAL constituent à cet égard des MCPAM particulièrement performants. Ils présentent de plus une remarquable recevabilité d'un point de vue sanitaire.

Diverses formulations d'ALCAL ayant une température de transition T dans les domaines précités sont données dans les exemples. Il est entendu que d'autres formulations appropriées seront aisément élaborées en se référant aux différents paramètres définis plus haut.

Si l'on reste dans le cas de Cn consécutifs (de même parité ou non), le niveau de température joue un rôle relativement décisif tant sur les possibilités de syncristallisation que sur le label δ et ce d'autant plus qu'il va de pair avec le niveau de $\varepsilon_n$. Ce coefficient est utilisé pour apprécier le degré d'homéomorphisme moléculaire $\varepsilon_k$.$\varepsilon_n$ est défini ainsi

$$\varepsilon_n = 1 - \frac{\Delta_n}{n_{minimum}}$$

où $\Delta_n$ est la différence $n_A$ - $n_B$ entre les 2 molécules $Cn_A$ et $Cn_B$ à comparer, et $n_{minimum}$ la valeur du n de la molécule la plus courte.

A basse température, on disposera surtout de possibilités intéressantes de mélanges eutectiques d'ALCAL (avec δ = 0°C), au fur-et-à mesure que le niveau de température T s'élèvera, les limites de syncristallisation augmenteront et les plages à haut label se feront plus importantes.

Si l'on choisit des Cn non consécutifs, les rôles des ΔT et des $\varepsilon_n$ joueront dans le même sens et s'ajouteront à l'effet niveau de T explicité ci-dessus. C'est ainsi qu'un écart important entre les nA et nZ, sera associé à des ΔT d'autant plus forts et des $\varepsilon_n$ d'autant plus faibles que le niveau T sera bas. A contrario, plus le niveau T sera élevé, plus on aura des ALCAL à δ recevables avec des n éloignés et donc plus grande sera la possibilité d'avoir des formulations différentes pour un $T_\delta$ requis car on pourra alors augmenter plus aisément le nombre de constituants.

Parmi les ALCAL, on citera ceux formés notamment de chaînes de C8 à C16, en particulier chacun de ces alcanes dopés, les binaires, les ternaires, les quaternaires ou plus, consécutifs ou non, le cas échéant également dopés, avec des proportions molaires telles que les valeurs $T_\delta$ correspondent strictement aux exigences requises.

Les applications évoquées plus haut seront réalisées avantageusement avec des ALCAL renfermant majoritairement des chaînes de plus de 14 atomes de carbone, le cas échéant dopés.

Dans le domaine paramédical, les applications sont également très diverses et couvrent notamment un domaine de température allant de -80°C à +75°C.

Des secteurs particulièrement concernés comprennent le conditionnement, la protection d'instruments, les manipulations isothermes ou à températures contrôlées, l'élaboration de vêtements isothermes, les insuffisances fonctionnelles et thérapies symptomatiques.

Pour les applications relatives au conditionnement et/ou au transport, avec non rupture de la chaîne de froid, on a avantageusement recours à des MCPAM recevables à une température donnée, dans un domaine allant de -80°C à +16°C environ.

Ainsi des MCPAM ayant un changement de phase à une température voisine de -80°C sont particulièrement utiles pour la conservation et/ou le transport de greffons d'os et/ou tendons, de plasma ou de sérum.

Des MCPAM recevables aux environs de -30°C permettent d'effectuer dans les meilleures conditions le transport de médicaments ou de plasma.

Il est particulièrement intéressant d'utiliser des MCPAM recevables autour de -20°C pour les banques d'os ou encore pour les transports post-mortem.

A des températures supérieures, les MCPAM allant de -10°C à +6°C environ offrent un grand intérêt. On les utilise ainsi avec avantage notamment pour le transport d'organes ou de membres amputés et pour la conservation de globules rouges ou d'organes.

Les MCPAM ayant des transitions de phase dans un domaine compris entre +6°C et +16°C présentent également un grand intérêt. Ils permettent notamment de conserver divers types de tissus ou cellules comme les greffons cornéens ou certains spermes. Ils permettent également la conservation des vaccins qui pourront être ainsi transportés et disponibles pour le patient au moment d'un accident.

Dans un domaine de température supérieur à +16°C environ, on utilise plus particulièrement les MCPAM pour le transport de médicaments.

D'autres applications dans le domaine paramédical se rapportent aux manipulations isothermes ou à température contrôlée.

Les MCPAM ayant une température de transition dans un domaine allant de -80°C à -10°C environ conviennent tout particulièrement en cryomicrotomie, par exemple de rachis ou d'articulations. Ceux qui possèdent une température de transition dans un domaine allant de -10°C à +6°C environ présentent une grande utilité pour la décongélation de plasma et de cellules, pour l'exploration hémodynamique, l'analyse des gaz du sang, pour les prélèvements stériles de tissus, par exemple de muscles ou de vaisseaux, et les cultures cellulaires.

Les MCPAM ayant des températures de transition plus élevées, notamment dans un domaine de température de +20°C et plus, sont utiles par ordre croissant de température, pour les boîtes de culture et les portoirs d'éprouvettes (de +20°C à +50°C), en électrophysiologie du nerf (entre +30°C et +35°C), pour le réchauffement de transplants, pour des essais enzymatiques (entre +35°C et +37°C) et pour le réchauffement du sang avant transfusion (vers +37°C).

D'autres applications dans le domaine paramédical concernent les insuffisances fonctionnelles et les thérapies symptomatiques.

Ainsi, les MCPAM obtenus selon l'invention, ayant une transition de phase

- dans un domaine de -10°C à +6°C environ, sont tout spécialement appropriés en soins pré- et post-opératoires, en particulier en ophtalmologie (entre 0°C et +6°C),

- dans un domaine de +6°C à +16°C environ, aux soins vétérinaires ou en cryothérapie par exemple pour constituer des bandages pour entorses, et

- dans un domaine de température supérieur à +16°C, en cryothérapie, pour obtenir des effets rafraîchissants et en particulier pour certains soins (autour de +35°C).

- pour la gamme supérieure à +37°C, ils seront utiles pour des couveuses ou encore pour le réchauffement local ou général de malades en cours d'opération ou en soins post-opératoires (couvertures, matelas, par exemple matelas avec résistance incorporée dans lesquels les MCPAM peuvent être associés à d'autres matériaux, comme des matériaux fibreux ou expansés pour des raisons de confort). Des couches thermiquement isolantes sur les faces externes, qui ne sont pas en contact avec le patient, prolongeront l'autonomie de fonctionnement.

Ils seront également utiles en calothérapie pour traiter par exemple des rhumatismes, ou réaliser des bouillotes à chaleur latente.

Pour faciliter l'utilisation en milieu familial, plus spécialement dans ce type d'application, on prévoit d'ajouter au MCPAM une faible dose de colorant, de qualité alimentaire afin de ne pas perturber la recevabilité sanitaire des matériaux. Cette disposition permet un repérage facile du niveau T de l'application (exemples : bleu pour T = +6°C, vert pour +25°C, incolore pour +35°C, jaune pour +39°C, orange pour +45°C et rouge pour +50°C). Les prescriptions d'utilisation de telle ou telle gamme de température viendront naturellement du corps médical.

On notera enfin que le fait de pouvoir disposer de MCPAM avec label $T_\delta$ , et ce, au niveau T souhaité, offre aux praticiens la possibilité d'effectuer des tests cliniques à diverses températures afin de dégager les plus pertinentes.

Dans ces applications dans le domaine paramédical, aux différents niveaux de température considérés, le recours à des MCPAM constitués par des ALCAL, le cas échéant comportant des groupes fonctionnels s'avère spécialement avantageux.

Les MCPAM permettent également de résoudre les problèmes de sécurité et/ou de protection de produits, d'installation et de locaux. Ces applications concernent un large domaine de température allant typiquement de -80°C à +200°C.

Ils constituent une solution de grand intérêt notamment pour

- le transport de produits chimiques inflammables et/ou dangereux, à basse température,

- la protection d'installations électriques, systèmes électroniques ou informatiques, où ils sont éventuellement couplés avec des systèmes d'alarme (les gammes de température pour ce type d'utilisation sont très variées, pouvant aller jusqu'à +200°C et plus, par exemple dans les cas d'incendies), une gamme de température particulièrement intéressante se situe entre +70°C et 90°C,

- contre les coupures de courant intempestives, ce qui évite le recours à des contrats très onéreux concernant la non rupture de courant dans des installations nécessitant un maintien isotherme, par exemple des mûrisseries de fruits ou légumes, ou des cuves de fermentations.

Les MCPAM sont également particulièrement utiles dans le domaine de la protection comme solution aux problèmes d'économie d'énergie. Ils constituent des moyens de protection de grande efficacité par exemple pour les cumulus, les bains chauds, apportant un confort supplémentaire, les réservoirs thermiques pour pompes à chaleur (créneau de +45°C à +55°C pour des sources chaudes, et de +3°C à +10°C pour des sources froides), les écrans thermiques au froid pour les grands ensembles (gamme de +4°C à +8°C), ou encore le chauffage domestique (par chaleur latente) pouvant être intégré comme élément de décoration (objets transparents ou non, statues par exemple).

En outre, ils peuvent être utilisés dans des cultures et en particulier assurent une protection des racines des plantes en serre, évitant le chauffage complet de la serre, ou dans des portoirs d'éprouvettes.

Dans des applications domestiques ou industrielles, les MCPAM seront utilisés avec avantage pour les articles à autonomie d'emploi à la maison ou à l'extérieur, tels que bigoudis, fers à repasser, biberons, en camping par exemple.

Ils permettent également de fabriquer aisément des gradients thermiques croissants ou décroissants qui peuvent être aussi forts ou aussi faibles que voulu et sont donc utilisables pour la régulation d'enceintes à gradient thermique telles que celles requises par exemple en croissance cristalline.

Dans les différentes applications évoquées ci-dessus, on a recours avec avantage, pour l'élaboration des MCPAM, à des ALCAL, substitués ou non, le cas échéant dopés, ainsi qu'aux composés cycliques et hétérocycliques comme défini plus haut. D'autres alliages moléculaires intéressants comprennent des chaînes hydrocarbonées, différentes des alcanes avec des groupements fonctionnels. D'autres alliages moléculaires encore sont formés de chaînes polymères.

Il va de soi que dans les diverses applications des MCPAM le mode d'utilisation peut être diversifié, un MCPAM pouvant être utilisé seul, associé à un ou plusieurs autres MCPAM et/ou MCP de façon à constituer des multicouches (ce qui est avantageusement utilisable pour former des gradients de température).

Dans de nombreuses applications, le MCPAM fonctionne en relais avec une source d'énergie.

D'une manière générale, l'invention fournit les moyens de fabriquer les matériaux les mieux adaptés pour une application donnée. Cette fabrication est d'autant plus facilitée que les matériaux sont maléables. Les compositions liquides sont ainsi parfaitement adaptées et sont solidifiées sur une forme appropriée.

Les MCPAM sont conditionnés dans des structures adaptées à leur utilisation.

Ces conditionnements comportent, d'une façon générale, une double paroi en un matériau à fort pouvoir d'isolation thermique, tout au moins en ce qui concerne la paroi en contact avec le milieu extérieur lorsque les deux parois sont élaborées à l'aide de matériaux différents.

Des matériaux appropriés comprennent le verre, les métaux, le matériau commercialisé sous la marque plastishield$^{R}$, à base de verre et de polymères, les matières plastiques et les polymères expansés à cellules closes.

A titre indicatif, on citera les polyoléfines, comme le polyéthylène haute densité (PEhd) ou basse densité (PEbd) ou encore le polypropylène (PP), les polyesters en particulier le polytéréphtalate d'éthylène ou des acrylates, les styrènes, comme le polystyrène (PSE) ou le polystyrène expansé (SE), ou l'acrylonitrile-butadiène-styrène (ABS) les polyamides (PA), les polyvinyles comme le polychlorure de vinyle (PVC), les polymères fluorés comme le polytetrafluoroéthylène ou PTFE.

Ces matériaux permettent de réaliser des surfaces lisses, ondulées, ou alvéolées.

Ils peuvent être le cas échéant renforcés par exemple par des entretoises pour assurer une tenue satisfaisante de la structure.

Pour les mettre en forme, on a recours aux techniques habituelles des plasturgistes. Ainsi pour réaliser des gourdes, bidons, bouteilles, flacons, pichets, tonnelets, futs, cuves, réservoirs, bacs, caisses, on utilise des méthodes d'injection, d'injection soufflage, d'injection soufflage par bi-orientation, d'extrusion soufflage, de soufflage ou de rotomoulage.

Des conditionnements tels que pots, gobelets, barquettes, cloches, plateaux repas, alvéolés ou non, avec armatures ou non, et le cas échéant des dispositifs de clipages, seront fabriqués par thermoformage ou injection.

Lorsque le conditionnement ne nécessite pas de double paroi, par exemple pour fabriquer des récipients du type pochettes, doses souples, cartouches, gaines, sacs, sachets, briquettes, on aura recours avantageusement aux techniques d'extrusion ou de calandrage.

Le remplissage du conditionnement par le MCPAM est effectué à partir de la composition liquide ou en poudre.

On pourra le cas échéant préformer l'alliage liquide placé dans une matrice en le solidifiant à l'azote liquide. Il pourra alors être transféré à l'état solide dans le conditionnement définitif.

L'invention sera illustrée ci-après, par des exemples de préparation d'alliages moléculaires et leurs applications comme MCPAM.

Dans ces exemples, il est fait référence aux figures 1 à 9, qui représentent les diagrammes donnant les labels $\delta$ de divers alliages moléculaires ternaires.

Caractérisation des matériaux :

1. Analyse RX (Guinier-Lenné, Guinier-Simon) pour préciser le nombre et la nature des phases en présence en

fonction de la température, mettre en évidence les changements de phases, et prouver, qu'avant la fusion les matériaux sont bien sous forme d'alliages (et non des mélanges de composés initiaux).

2. Analyse calorimétrique différentielle (AED, DSC) : pour la détermination précise des températures pertinentes et des enthalpies associées, en respectant les deux exigences suivantes :

- étalonnage préalable effectué strictement dans les mêmes conditions expérimentales que celles des analyses,

- exploitation des signaux AED ou DSC par la "méthode des facteurs de forme" mise au point par Haget et al, Calorim. Anal. Therm. (1987), 18, 255, et Courchinoux et al., J. Chim. Phys. (1989), 86, 3, 561.

Les exemples qui suivent numérotés 1 à 5 se rapportent aux ALCAL, les exemples 6 à 9 à des alliages moléculaires contenant ou formés de chaînes à fonction acide et les exemples 10 à 12 à des applications des alliages moléculaires élaborés selon de l'invention. (On notera que les valeurs de $T_\delta$ et de $\delta$ données dans ces exemples s'entendent T et $\delta$ en °C).
Les exemples 1 à 7 concernent respectivement :

- exemple 1 : des formulations d'ALCAL dopés,

- exemple 2 : des formulations d'ALCAL binaires dopés,

- exemple 3 : des formulations d'ALCAL ternaires et plus dopés,

- exemple 4 : l'étude de l'évolution d'ALCAL binaires,

  . ALCAL binaires de même parité [1) consécutifs ; 2) non consécutifs],

  . ALCAL binaires avec parité différente [1) consécutifs ; 2) non consécutifs],

- exemple 5 : des ALCAL ternaires :

  a) étude de l'évolution d'ALCAL [1) consécutifs, 2) non directement consécutifs],

  b) diverses formulations d'ALCAL ternaires (1 à 9)

**. Remarques concernant les alcanes.**

Lorsque $8 \leq n \leq 20$, les formes avant fusion des alcanes à n impairs (Cni) sont de type hexagonal et ceux à n pairs (Cnp) de type triclinique.
Lorsque $21 \leq n \leq 43$, tous les alcanes sont de type hexagonal avant la fusion.
Enfin lorsque $n \geq 44$, tous les alcanes sont orthorhombiques avant la fusion.

**. Méthode générale de préparation.**

Lorsqu'on utilise des alcanes tous solides, ou certains d'entre eux solides, on dissoud tout d'abord les alcanes de départ, selon les proportions appropriées, dans un solvant commun tel que l'éther, puis on évapore le solvant, de préférence sous courant de gaz inerte tel que l'azote. En variante, on soumet les produits à une fusion en agitant, pour avoir un mélange homogène, puis à une trempe.

**Exemple 1 : Formulations d'alcanes dopés.**

On rapporte dans le tableau 1 qui suit des formulations d'alcanes dopés. Pour chaque formulation d'alliage, on indique la température de transition T en °C, la fenêtre thermique 6 en °C, et la variation d'enthalpie $\Delta H$ en J/g.

Tableau 1

| Cn | Formulations | T(°C) | δ(°C) | ΔH(J/g) |
|---|---|---|---|---|
| C8 | C80,980 C100,020 | -57,2 | 1,4 | 170 |

Tableau 1 (suite)

| Cn | Formulations | T(°C) | δ(°C) | ΔH(J/g) |
|---|---|---|---|---|
| C9 | $C9_{0,995}$ $C10_{0,005}$ | -53,0 | 1,3 | 117 |
| C10 | $C10_{0,990}$ $C11_{0,010}$ | -29,0 | 1,2 | 196 |
| C11 | $C11_{0,998}$ $C12_{0,001}$ $C13_{0,001}$ | -25,0 | 1,3 | 141 |
| C12 | $C12_{0,993}$ $C11_{0,004}$ $C13_{0,004}$ | -9,4 | 1,2 | 210 |
| C13 | $C13_{0,998}$ $C11_{0,001}$ $C12_{0,001}$ | -4,7 | 0,6 | 162 |
| C14 | $C14_{0,999}$ $C13_{0,001}$ | +5,9 | 1,5 | 216 |
| C15 | $C15_{0,994}$ $C14_{0,001}$ $C16_{0,005}$ | +10,0 | 0,6 | 159 |
| C16 | $C16_{0,996}$ $C15_{0,003}$ $C17_{0,001}$ | +17,9 | 1,0 | 227 |
| C17 | $C17_{0,998}$ $C15_{0,001}$ $C16_{0,001}$ | +22,2 | 0,5 | 168 |
| C18 | C180,994 C190,006 | +28,4 | 0,9 | 232 |
| C19 | $C19_{0,980}$ $C20_{0,020}$ | +32,3 | 0,9 | 168 |
| C20 | $C20_{0,980}$ $C19_{0,020}$ | +36,4 | 5,0 | 240 |
| C20 | $C20_{0,994}$ $C19_{0,006}$ | +36,5 | 1,6 | 241 |
| C21 | $C21_{0,997}$ $C23_{0,003}$ | +39,9 | 0,3 | 156 |
| C22 | $C22_{0,980}$ $C21_{0,020}$ | +43,3 | 0,9 | 152 |
| C22 | $C22_{0,997}$ $C21_{0,003}$ | +43,8 | 0,9 | 152 |
| C23 | $C23_{0,996}$ $C22_{0,002}$ $C24_{0,002}$ | +47,5 | 0,2 | 158 |
| C23 | $C23_{0,980}$ $C24_{0,020}$ | +48,2 | 0,8 | 160 |
| C24 | $C24_{0,950}$ $C22_{0,050}$ | +50,1 | 0,2 | 157 |
| C24 | $C24_{0,930}$ $C20_{0,050}$ $C22_{0,020}$ | +50,5 | 1,1 | 158 |
| C24 | $C24_{0,950}$ $C26_{0,050}$ | +50,9 | 0,2 | 158 |
| C25 | $C25_{0,992}$ $C24_{0,005}$ $C26_{0,003}$ | +53,8 | 0,2 | 161 |
| C26 | $C26_{0,998}$ $C24_{0,002}$ | +56,9 | 0,1 | 161 |
| C26 | $C26_{0,999}$ $C25_{0,001}$ | +56,9 | 0,1 | 161 |

On remarquera à l'examen de ce tableau que les différentes formulations dopées étudiées ont une transition sur un intervalle étroit de température, toujours inférieur à 2°C, excepté pour une formulation, et même ne dépassant pas 1°C pour la majorité, les ΔH étant pour la plupart supérieurs à 150 J/g et même dépassant pour certains 200 J/g.

**<u>Exemple 2</u> : Formulations d'ALCAL binaires dopés.**

On rapporte dans le tableau 2 suivant les caractéristiques d'alliages binaires dopés.

Tableau 2

| Exemples : | | | | |
|---|---|---|---|---|
| Cn-Cn | Formulation | T(°C) | δ(°C) | ΔH(J/g) |
| C8-C10 | C80,930 C100,060 C90,010 | -57,7 | 2,8 | 170 |
| C10-C11 | $C10_{0,690}$ $C11_{0,300}$ $C12_{0,010}$ | -35,9 | 2,1 | 137 |
| C10-C11 | $C10_{0,490}$ $C11_{0,490}$ $C12_{0,020}$ | -33,2 | 4,2 | 129 |
| C10-C11 | $C10_{0,310}$ $C11_{0,680}$ $C12_{0,010}$ | -30,4 | 4,2 | 125 |
| C11-C12 | $C11_{0,880}$ $C12_{0,100}$ $C13_{0,020}$ | -24,9 | 1,1 | 141 |
| C11-C12 | $C11_{0,490}$ $C12_{0,490}$ $C13_{0,020}$ | -21,6 | 2,0 | 134 |
| C12-C13 | $C12_{0,690}$ $C13_{0,300}$ $C11_{0,010}$ | -13,5 | 2,7 | 146 |
| C12-C13 | $C12_{0,480}$ $C13_{0,480}$ $C11_{0,040}$ | -11,8 | 2,2 | 146 |
| C12-C13 | $C12_{0,200}$ $C13_{0,780}$ $C11_{0,020}$ | -8,0 | 2,4 | 150 |
| C13-C15 | $C13_{0,780}$ $C15_{0,200}$ $C14_{0,020}$ | -5,0 | 1,0 | 144 |
| C13-C14 | $C13_{0,690}$ $C14_{0,300}$ $C15_{0,010}$ | -3,9 | 1,4 | 152 |
| C13-C14 | $C13_{0,485}$ $C14_{0,490}$ $C15_{0,025}$ | -2,5 | 1,8 | 152 |

Tableau 2 (suite)

| Exemples : | | | | |
|---|---|---|---|---|
| Cn-Cn | Formulation | T(°C) | δ(°C) | ΔH(J/g) |
| C13-C15 | $C13_{0,530}$ $C15_{0,450}$ $C14_{0,020}$ | 0 | 7,0 | 148*) |
| C14-C16 | $C14_{0,790}$ $C16_{0,200}$ $C15_{0,010}$ | +2,9 | 2,0 | 159 |
| C14-C16 | C140,890 $C16_{0,100}$ $C15_{0,010}$ | +3,2 | 1,9 | 164 |
| C14-C15 | $C14_{0,400}$ $C15_{0,570}$ $C13_{0,030}$ | +5,2 | 3,9 | 147 |
| C13-C15 | $C13_{0,200}$ $C15_{0,790}$ $C14_{0,010}$ | +6,0 | 8,0 | 159*) |
| C14-C15 | $C14_{0,260}$ $C15_{0,700}$ $C16_{0,040}$ | +7,7 | 2,0 | 155 |
| C14-C16 | $C14_{0,410}$ $C16_{0,580}$ $C15_{0,010}$ | +8,8 | 4,9 | 148 |
| C15-C16 | $C15_{0,840}$ $C16_{0,130}$ $C14_{0,030}$ | +9,5 | 1,0 | 153 |
| C15-C16 | $C15_{0,840}$ $C16_{0,150}$ $C14_{0,010}$ | +10,5 | 0,8 | 150 |
| C15-C16 | $C15_{0,640}$ $C16_{0,330}$ $C14_{0,030}$ | +10,7 | 2,0 | 152 |
| C15-C16 | $C15_{0,690}$ $C16_{0,300}$ $C14_{0,010}$ | +11,2 | 1,3 | 156 |
| C15-C17 | C150,685C170,300 $C16_{0,015}$ | +11,3 | 1,3 | 146 |
| C15-C16 | C150,490 $C16_{0,500}$ $C14_{0,010}$ | +12,3 | 1,7 | 157 |
| C15-C17 | $C15_{0,480}$ $C17_{0,500}$ $C16_{0,020}$ | +14,0 | 3,0 | 150 |
| C14-C16 | $C14_{0,100}$ $C16_{0,890}$ $C15_{0,010}$ | +16,0 | 3,2 | 175 |
| C15-C17 | $C15_{0,300}$ $C17_{0,690}$ $C16_{0,010}$ | +17,0 | 4,4 | 154 |
| C16-C18 | C160,690 C180,290 C170,020 | +18,0 | 1,9 | 170 |
| C16-C18 | C160,550 C180,440 C170,010 | +19,8 | 2,0 | 165 |
| C16-C17 | C160,300 $C17_{0,685}C15_{0,010}C18_{0,005}$ | +19,8 | 1,5 | 160 |
| C15-C17 | C150,100 C170,890 C160,010 | +20,4 | 2,9 | 158 |
| C16-C17 | $C16_{0,100}$ $C17_{0,885}C15_{0,005}C18_{0,010}$ | +21,3 | 1,1 | 165 |
| C17-C18 | C170,300 C180,690 C190,010 | +25,7 | 1,2 | 159 |
| C18-C22 | $C18_{0,870}$ $C22_{0,100}$ $C20_{0,030}$ | +27,8 | 1,8 | 164 |
| C18-C19 | C180,800 C190,170 $C17_{0,030}$ | +27,9 | 1,5 | 156 |
| C18-C20 | C180,780 C200,200 $C19_{0,020}$ | +28,3 | 1,8 | 155 |
| C18-C19 | $C18_{0,780}$ $C19_{0,200}$ $C20_{0,020}$ | +28,4 | 1,5 | 155 |
| C18-C20 | C180,780 C200,200 C220,020 | +28,5 | 2,0 | 154 |
| C18-C19 | C180,580 C190,400 C170,020 | +28,8 | 1,6 | 156 |
| C18-C19 | $C18_{0,580}$ $C19_{0,390}$ $C20_{0,030}$ | +29,4 | 1,6 | 156 |
| C18-C22 | C180,700 C220,280 C200,020 | +30,8 | 3,9 | 139 |
| C18-C20 | $C18_{0,540}$ $C20_{0,450}$ $C19_{0,010}$ | +30,8 | 1,9 | 146 |
| C18-C20 | $C18_{0,530}$ C200,440 C220,030 | +30,9 | 2,0 | 146 |
| C18-C20 | C180,190 C200,800 C220,010 | +33,9 | 2,0 | 150 |
| C18-C20 | C180,200 C200,790 $C19_{0,010}$ | +33,9 | 2,0 | 150 |
| C19-C20 | C190,300 C200,680 C210,020 | +35,2 | 0,9 | 151 |
| C19-C21 | C190,290 C210,690 C200,020 | +35,5 | 1,2 | 151 |
| C20-C22 | C200,770 $C22_{0,200}$ C180,030 | +37,5 | 0,9 | 146 |
| C20-C22 | $C20_{0,794}C22_{0,200}$ $C19_{0,004}C21_{0,002}$ | +37,6 | 0,8 | 146 |
| C20-C22 | C200,800 C220,170 C210,030 | +37,8 | 0,8 | 146 |
| C20-C22 | C200,780 C220,200 C240,020 | +38,0 | 0,8 | 145 |
| C20-C24 | C200,870 $C24_{0,110}$ C220,020 | +38,6 | 1,2 | 145 |
| C20-C22 | C200,480 $C22_{0,490}$ C210,030 | +39,7 | 1,5 | 148 |
| C20-C22 | $C20_{0,471}C22_{0,524}C19_{0,003}C21_{0,002}$ | +39,8 | 1,3 | 148 |
| C20-C22 | $C20_{0,500}$ $C22_{0,480}$ $C24_{0,020}$ | +40,0 | 1,5 | 147 |
| C20-C22 | $C20_{0,400}$ $C22_{0,580}$ $C18_{0,020}$ | +40,1 | 1,5 | 147 |
| C20-C22 | $C20_{0,376}C22_{0,620}$ $C19_{0,002}C21_{0,002}$ | +40,3 | 1,2 | 147 |
| C20-C22 | $C20_{0,380}C22_{0,600}C24_{0,020}$ | +40,6 | 1,4 | 147 |

(*) comparatifs

Tableau 2 (suite)

| Exemples : | | | | |
|---|---|---|---|---|
| Cn-Cn | Formulation | T(°C) | δ(°C) | ΔH(J/g) |
| C20-C22 | $C20_{0,300}C22_{0,690}C21_{0,010}$ | +41,2 | 1,4 | 147 |
| C20-C22 | $C20_{0,279}C22_{0,717}C19_{0,002}C21_{0,002}$ | +41,3 | 1,2 | 147 |
| C20-C22 | $C20_{0,180}C22_{0,800}C18_{0,020}$ | +41,8 | 1,1 | 147 |
| C21-C22 | $C21_{0,490}C22_{0,490}C23_{0,020}$ | +41,8 | 0,6 | 150 |
| C20-C22 | $C20_{0,184}C22_{0,812}C19_{0,001}C21_{0,003}$ | +42,0 | 0,9 | 147 |
| C20-C22 | $C20_{0,090}C22_{0,900}C21_{0,010}$ | +42,8 | 0,9 | 150 |
| C20-C22 | $C20_{0,093}C22_{0,903}C19_{0,001}C21_{0,003}$ | +42,9 | 1,0 | 150 |
| C21-C23 | C210,580 C230,400 C220,020 | +43,3 | 1,3 | 152 |
| C22-C24 | C220,700 C240,280 C200,020 | +45,2 | 0,8 | 153 |
| C22-C23 | C220,490 $C23_{0,500}$ C240,010 | +45,7 | 0,5 | 155 |
| C22-C24 | C220,700 C240,280 C230,020 | +45,7 | 0,9 | 152 |
| C22-C24 | C220,680 C240,300 C260,020 | +45,7 | 0,8 | 153 |
| C22-C24 | C220,300 $C24_{0,680}$ $C20_{0,020}$ | +48,1 | 0,9 | 155 |
| C22-C24 | C220,280 $C24_{0,700}$ C230,020 | +48,4 | 0,9 | 156 |
| C22-C24 | C220,300 $C24_{0,680}$ C260,020 | +48,6 | 0,9 | 155 |
| C23-C24 | C230,470 $C24_{0,500}$ C220,030 | +48,9 | 0,5 | 158 |
| C22-C24 | $C22_{0,050}$ $C24_{0,930}$ $C23_{0,020}$ | +50,2 | 0,4 | 158 |
| C22-C26 | C220,500 $C26_{0,490}$ C240,010 | +50,2 | 2,6 | 157 |
| C24-C26 | C240,900 $C26_{0,080}$ C200,020 | +50,2 | 0,4 | 158 |
| C24-C25 | $C24_{0,500}$ $C25_{0,490}$ $C26_{0,010}$ | +51,7 | 0,4 | 159 |
| C24-C26 | C240,490 $C26_{0,500}$ C200,010 | +52,2 | 0,9 | 158 |
| C23-C25 | $C23_{0,100}$ $C25_{0,890}$ $C24_{0,010}$ | +53,0 | 0,9 | 161 |
| C24-C26 | C240,490 $C26_{0,500}$ $C22_{0,010}$ | +53,5 | 0,9 | 162 |
| C26-C32 | C260,800 $C32_{0,190}$ C300,010 | +57,3 | 2,0 | 160 |
| C28-C32 | $C28_{0,790}C32_{0,200}C30_{0,010}$ | +62,4 | 0,4 | 160 |
| C30-C32 | $C30_{0,100}C32_{0,880}C31_{0,010}C33_{0,010}$ | +66,2 | 0,5 | 159 |
| C35-C36 | C350,890 C360,090 C370,020 | +75,2 | 0,4 | 160 |
| C44-C50 | $C44_{0,765}C50_{0,210}C46_{0,010}C48_{0,015}$ | +86,7 | 0,7 | 223 |
| C44-C50 | $C44_{0,490}C50_{0,485}C46_{0,005}C48_{0,020}$ | +87,4 | 0,5 | 220 |
| C44-C50 | $C44_{0,395}C50_{0,585}C46_{0,005}C48_{0,015}$ | +89,2 | 1,5 | 225 |
| C46-C50 | $C46_{0,100}$ $C50_{0,890}$ C440,010 | +91,9 | 0,6 | 215 |

**Exemple 3 : Formulations d'ALCAL ternaires, et plus, dopés.**

Le tableau 3 ci-après comporte les caractéristiques d'alliages ternaires dopés ou d'alliages supérieurs.

Tableau 3 :

| Alcanes | Formulation | T(°C) | δ | ΔH(J/g) |
|---|---|---|---|---|
| C12-C13-C14 | C12 0,130 C13 0,607 C14 0,262 C11 0,001 | -6,2 | 4,8 | 130 |
| C15-C16-C17 | C15 0,660 C16 0,240 C17 0,070 C18 0,030 | +11,3 | 2,0 | 152 |
| C14-C15-C16 | C14 0,214 C15 0,226 C16 0,558 C13 0,001 C17 0,001 | +8,5 | 2,3 | 152 |
| C14-C15-C16 | C14 0,350 C15 0,430 C16 0,160 C17 0,040 C18 0,020 | +7,1 | 2,7 | 146 |
| C14-C15-C16 | C14 0,320 C15 0,380 C16 0,250 C17 0,040 C18 0,010 | +7,5 | 2,5 | 144 |
| C14-C15-C16 | C14 0,280 C15 0,350 C16 0,320 C17 0,040 C18 0,010 | +8,3 | 3,0 | 143 |
| C14-C15-C16-C18 | C14 0,310 C15 0,370 C16 0,140 C17 0,040 C18 0,140 | +7,5 | 3,4 | 127 |
| C14-C15-C16-C17 | C14 0,290 C15 0,350 C16 0,130 C17 0,130 C18 0,010 | +8,6 | 4,1 | 146 |
| C15-C16-C17 | C15 0,366 C16 0,462 C17 0,167 C18 0,005 | +13,5 | 2,0 | 154 |
| C15-C16-C17 | C15 0,272 C16 0,069 C17 0,649 C18 0,010 | +17,3 | 3,9 | 150 |
| C15-C16-C17 | C15 0,065 C16 0,067 C17 0,848 C18 0,020 | +20,6 | 2,8 | 160 |
| C18-C20-C22 | C18 0,850 C20 0,065 C22 0,065 C16 0,020 | +27,5 | 2,0 | 150 |
| C18-C20-C22 | C18 0,625 C20 0,230 C22 0,130 C24 0,015 | +30,0 | 1,9 | 142 |
| C18-C20-C22 | C18 0,330 C20 0,310 C22 0,330 C24 0,030 | +36,0 | 3,7 | 139 |
| C20-C22-C26 | C20 0,620 C22 0,230 C26 0,130 C24 0,020 | +39,5 | 1,8 | 143 |
| C20-C22-C24 | C20 0,340 C22 0,320 C24 0,310 C23 0,030 | +43,4 | 2,8 | 147 |
| C22-C24-C26 | C22 0,450 C24 0,360 C26 0,160 C25 0,020 C27 0,010 | +47,7 | 1,1 | 154 |
| C22-C24-C26 | C22 0,170 C24 0,640 C26 0,160 C25 0,030 | +50,3 | 0,8 | 156 |
| C44-C46-C48-C50 | C44 0,350 C46 0,350 C48 0,080 C50 0,200 C47 0,020 | +86,9 | 0,6 | 220 |
| C44-C46-C50 | C44 0,390 C46 0,390 C50 0,190 C48 0,030 | +87,2 | 0,5 | 224 |

**Exemple 4 : ALCAL binaires. Etude de l'évolution**

Dans cet exemple et les suivants, le degré d'homéomorphisme moléculaire $\varepsilon_k$ est apprécié par le coefficient $\varepsilon_n$ défini plus haut :

où $\Delta n$ est la différence $n_A$ - $n_B$ entre les 2 molécules $Cn_A$ et $Cn_B$ à comparer, et $n_{minimum}$ la valeur du n de la molécule la plus courte.

I ALCAL binaires de même parité :

A et Z sont tous deux pris dans les alcanes pairs ou tous deux pris dans les alcanes impairs.

1 ALCAL consécutifs

Ils sont de type Cnp-C(np + 2) ou de type Cni-C(ni + 2). Les deux types ont des comportements très semblables.

On obtient des compositions formant des MCPAM intéressants pour $C8_{1-x}$ $C10_x$ avec $x \leq 0{,}15$, notamment avec,

x = 0,05 (T = -57,5°C, δ = 2,8, et ΔH = 170 J/g)
x = 0,11 (T = -59,3°C, δ = 1,6, ΔH = 180 J/g).

C10-C12 : $\varepsilon_n$ = 0,80, ΔT = 20,1°C : on forme un eutectique pour la composition globale 80 % C10 + 20 % C12 (T = -38°C, δ = 0,ΔH = 195 J/g).

2ème cas : $12 \leq n \leq 18$ ; on a alors $0{,}80 < \varepsilon_n \leq 0{,}88$ et $10 \leq \Delta T < 20$

Plus n va augmenter, plus la miscibilité va s'étendre (en domaine de concentration) et plus les fuseaux vont devenir étroits.

C12-C14 : $\varepsilon_n$ = 0,83 ; ΔT = 15,5°C

la miscibilité s'étend mais les fuseaux sont larges.

Des alliages satisfaisants sont obtenus avec des constituants purs et surtout du côté C12. (Dans ce qui suit, les fenêtres thermiques δ concernant un domaine de concentration sont données avec un label global pris égal à un nombre entier : 1 quand δ ≤ 1, 2 quand 1 < δ ≤ 2, 4 quand 2 < δ ≤ 4. En revanche pour les exemples particuliers δ est donné avec sa valeur réelle).

Description des alliages $C12_{1-x}$ $C14_x$ recevables selon l'invention, avec
0 ≤ x < 0,05 : δ = 2
0,05 ≤ x < 0,15 : δ = 4,
par exemple, x = 0,11 : T = -12,0°C, δ = 3,1, ΔH = 175 J/g
0,15 ≤ x ≤ 0,22 : δ = 2,
par exemple, x = 0,19 : T = -14,2°C, δ = 1,7, ΔH = 159 J/g
0,92 ≤ x ≤1 : δ = 4,
par exemple, x = 0,95 : T = +5,0°C, δ = 3,6, ΔH = 205 J/g

C13-C15 : $\varepsilon_n$ = 0,85 ; ΔT = 15,5°C,

on observe une miscibilité en toutes proportions mais des fuseaux larges excepté du côté C13.

Description des alliages $C13_{1-x}$ $C15_x$ :
0 ≤ x < 0,20 : δ = 1
0,20 ≤ x < 0,30 : δ = 2,
par exemple x = 0,200 : T = -5,1°C, δ = 0,9, ΔH = 144 J/g 0,30 ≤ x < 0,40 : δ = 4
0,40 ≤ x < 0,95 : δ > 6, (*)
par exemple, x = 0,464 : T = -0,5°C, δ = 6,0, ΔH = 148 J/g
x = 0,794 : T = +6,1°C, δ = 7,8, ΔH = 159 J/g (*)
0,95 ≤ x ≤ 1 : δ = 4
avec dopage :

(*) comparatifs

| | |
|---|---|
| $C13_{0,78}$ $C15_{0,20}$ $C14_{0,02}$ | T = -5,0°C, δ = 1,0, ΔH = 144 J/g |
| $C13_{0,53}$ $C15_{0,45}$ $C14_{0,02}$ | T = 0,0°C, δ = 7,0, ΔH = 148 J/g (*) |

(*) comparatifs

C14-C16 : $\varepsilon_n$ = 0,86, ΔT = 12,3°C,

on observe une miscibilité totale lorsqu'on mélange les chaînes quelles que soient les proportions. La largeur du fuseau varie avec les zones.

Parmi les alliages $C14_{1-x}$ $C16_x$, on rapporte les résultats obtenus avec diverses valeurs de x.
0 ≤ x < 0,30 : δ = 2
par exemple, x = 0,10 : T = +3,2°C, δ = 1,9, ΔH=164 J/g
x = 0,20 : T = +2,9°C, δ=2 ΔH = 147 J/g
0,30 ≤ x < 0,47 : δ = 4
par exemple, x = 0,35 : T = +4,7°C, δ=2,8, ΔH = 137 J/g 0,47 ≤ x < 0,80 : δ= 6
par exemple, x = 0,60 : T = +9,0°C, δ=4,9, ΔH 148 J/g 0,80 ≤ x < 0,95 : δ = 4
par exemple, x = 0,90 : T = +16,1°C, δ=3,2, ΔH=176 J/g 0,95 ≤ x ≤1 : δ = 2

**Formulations de C14-C16 dopés :**

| | | | |
|---|---|---|---|
| $C14_{0,790}$ $C16_{0,200}$ $C15_{0,010}$ | T= +2,9°C, | δ = 2,0, | ΔH = 159 J/g |
| $C14_{0,890}$ $C16_{0,100}$ $C15_{0,010}$ | T= +3,2°C, | δ = 1,9, | ΔH = 164 J/g |
| $C14_{0,410}$ $C16_{0,580}$ $C15_{0,010}$ | T= +8,8°C, | δ = 4,9, | ΔH = 148 J/g |
| $C14_{0,100}$ $C16_{0,890}$ $C15_{0,010}$ | T=+16,0°C, | δ = 3,2, | ΔH = 175 J/g |

C15-C17 : $\varepsilon_n$ = 0,57 ; ΔT = 12,0°C

La miscibilité est totale pour $C15_{1-x}$ $C17_x$, mais δ est plus large au centre droit.
0 ≤ x < 0,20 δ = 1
0,20 ≤ x < 0,425 δ = 2
par exemple, x = 0,30 : T = +11,3°C, δ = 1,2, ΔH = 146 J/g 0,425 ≤ x < 0,57 δ = 4
par exemple, x = 0,50 : T = +14,1°C, δ = 2,9, ΔH = 156 J/g 0,57 ≤ x < 0,80 δ = 6
par exemple, x = 0,70 : T = +17,0°C, δ = 4,4, ΔH = 154 J/g 0,80 ≤ x < 0,95 δ = 4
par exemple, x = 0,90 : T = +20,2°C, δ = 2,9, ΔH = 158 J/g 0,95 ≤ x ≤ 1 δ = 2

Formulations dopées :

| | | | |
|---|---|---|---|
| $C15_{0,685}C17_{0,300}C16_{0,015}$ | T = +11,3°C, | δ = 1,3, | ΔH= 146 J/g |
| $C15_{0,480}C17_{0,500}C16_{0,020}$ | T = +14,0°C, | δ = 3,0, | ΔH= 150 J/g |
| $C15_{0,300}C17_{0,690}C16_{0,010}$ | T = +17,0°C, | δ = 4,4, | ΔH= 154 J/g |
| $C15_{0,100}C17_{0,890}C16_{0,010}$ | T = +20,4°C, | δ = 2,9, | ΔH= 158 J/g |

C16-C18 : $\varepsilon_n$ = 0,88 ; ΔT = 10,0°C

Toutes les formulations $C16_{1-x}$ $C18_x$ ont des δ ≤ 4. Les restrictions pour 0,03 < x < 0,10 et 0,75 < x < 0,97 correspondent à des élargissements du fuseau solidus-liquidus liés à des interférences avec des fuseaux solide-solide (ce phénomène apparaît dans plusieurs autres alliages binaires soit que les alcanes purs présentent eux-mêmes une transition, soit que celle-ci soit induite par l'autre Cn, comme c'est le cas dans ce binaire)
0,10 ≤ x < 0,50 : δ = 2
0,50 ≤ x ≤ 0,75 : δ = 4

Formulations dopées :

| | |
|---|---|
| $C16_{0,690}C18_{0,290}C17_{0,020}$ | T = +18,0°C, δ = 1,9, ΔH = 170 J/g |
| $C16_{0,550}C18_{0,440}C17_{0,010}$ | T = +19,8°C, δ = 2,0, ΔH = 165 J/g |

3ème cas :

18 ≤ n ≤ 22 ; on a alors 0,88 < $\varepsilon_n$ ≤0,90 et 7°C <ΔT< 10°C La miscibilité est totale, tous les alliages moléculaires ont des δ ≤ 2 seule est ressentie une très faible perturbation due à la transition éventuelle solide-solide (du C20 en particulier)

C18-C20 : $\varepsilon_n$ = 0,89 ; ΔT = 8,6°C

Les résultats suivants ont été obtenus avec $C18_{1-x}$ $C20_x$ :
0 ≤ x < 0,03 (dopage) : δ=1
0,03 ≤ x < 0,05 avec effet de transition de C18
0,05 ≤ x < 0,90 : 6=2
par exemple :

| | | | |
|---|---|---|---|
| x=0,20 | T = +28,2°C | δ=1,6 | ΔH=154 J/g |
| x=0,45 | T = +30,6°C | δ=1,8 | ΔH=146 J/g |
| x=0,80 | T = +33,7°C | δ=2,0 | ΔH=150 J/g |

0,90 ≤ x < 0,97 avec effet de transition de C20
0,97 ≤ x ≤ 1 : δ = 1

Avec des binaires dopés, les résultats suivants ont été obtenus :

| | | |
|---|---|---|
| $C18_{0,78}$ $C20_{0,20}$ $C19_{0,02}$ | T = +28,3°C | δ=1,8 |
| $C18_{0,78}$ $C20_{0,20}$ $C22_{0,02}$ | T = +28,5°C | δ=2,0 |
| $C18_{0,54}$ $C20_{0,45}$ $C19_{0,01}$ | T = +30,8°C | δ=1,9 |
| $C18_{0,53}$ $C20_{0,44}$ $C22_{0,03}$ | T = +30,9°C | 6=2,0 |
| $C18_{0,19}$ $C20_{0,80}$ $C22_{0,01}$ | T = +33,9°C | δ=2,0 |
| $C18_{0,20}$ $C20_{0,79}$ $C19_{0,01}$ | T = +33,9°C | δ=2,0 |

On constate donc que les binaires C18-C20 dopés ou non, répondent sensiblement de la même manière. Ainsi, le dopage d'un MCPAM donné permet d'ajuster T sans affecter δ (ni ΔH) pour une valeur de x.

C19-C21 : $\varepsilon_n$ = 0,89 ; ΔT = 8,4°C

$C19_{1-x}$ C21x
0 ≤ x < 0,20 : δ = 1
0,20 ≤ x < 0,90 : δ = 2
0,90 ≤ x ≤ 1 : δ = 1

Avec dopage (exemple)

| | |
|---|---|
| $C19_{0,290}$ $C21_{0,690}$ $C20_{0,020}$ | T = +35,5°C, δ = 1,2 ΔH=151 J/g |

C20-C22 : $\varepsilon_n$ = 0,90, ΔT = 7,6°C

On constate que le fuseau s'amincit par rapport à ceux des alliages précédents. On n'observe d'effet de transition solide-solide de C20 qu'entre 0,02<x<0,05.

Avec,
x < 0,40, on obtient δ = 1,
0,40 ≤ x < 0,75 : δ = 2

$0,75 \leq x \leq 1 : \delta = 1$

Exemples de formulations dopées, classées selon T (les ΔH sont pratiquement égaux à 147 J/g)

| | | |
|---|---|---|
| $C20_{0,794}$ $C22_{0,200}$ $C19_{0,004}$ $C21_{0,002}$ | T = +37,6°C | δ=0,8 |
| $C20_{0,80}$ $C22_{0,17}$ $C21_{0,03}$ | T = +37,8°C | δ=0,8 |
| $C20_{0,78}$ $C22_{0,20}$ $C24_{0,02}$ | T = +38,0°C | δ=0,8 |
| $C20_{0,48}$ $C22_{0,49}$ $C21_{0,03}$ | T = +39,7°C | δ=1,5 |
| $C19_{0,003}$ $C20_{0,471}$ $C21_{0,002}$ $C22_{0,524}$ | T = +39,8°C | δ=1,3 |
| $C20_{0,50}$ $C22_{0,48}$ $C24_{0,02}$ | T = +40,0°C | δ=1,5 |
| $C19_{0,002}$ $C20_{0,376}$ $C21_{0,002}$ $C22_{0,620}$ | T = +40,3°C | δ=1,2 |
| $C20_{0,38}$ $C22_{0,60}$ $C24_{0,02}$ | T = +40,6°C | δ=1,4 |
| $C20_{0,30}$ $C22_{0,69}$ $C21_{0,01}$ | T = +41,2°C | δ=1,4 |
| $C19_{0,002}$ $C20_{0,279}$ $C21_{0,002}$ $C22_{0,717}$ | T = +41,3°C | δ=1,2 |
| $C19_{0,001}$ $C20_{0,184}$ $C21_{0,003}$ $C22_{0,812}$ | T = +42,0°C | δ=0,9 |
| $C20_{0,09}$ $C22_{0,90}$ $C21_{0,01}$ | T = +42,8°C | δ=0,9 |
| $C19_{0,001}$ $C20_{0,093}$ $C21_{0,003}$ $C22_{0,903}$ | T = +42,9°C | δ=1,0 |

C21-C23 : $\varepsilon_n = 0,90$ ; ΔT = 7,1°C

$C21_{1-x}$ $C23_x$

$0 \leq x \leq 0,35 : \delta = 1$

$0,35 < x < 0,60 : \delta = 2$

$0,60 \leq x \leq 1 : \delta = 1$

Formulation dopée :

| | | | |
|---|---|---|---|
| $C21_{0,580}C23_{0,400}C22_{0,020}$ | T= 43,3°C, | δ = 1,3, | ΔH = 152 J/g |

4ème cas : $n \geq 22$ $\varepsilon_n > 0,90$ $\Delta T < 7°C$

A partir de C22-C24 inclus tous les binaires ont des miscibilités totales avec δ < 1 quel que soit x. Il en est de même pour les binaires dopés. On rapportera ci-après quelques exemples de dopés, des exemples complémentaires étant donnés dans le tableau 2

C22-C24 : $\varepsilon_n = 0,91$ ; ΔT = 6,5°C

| | | | |
|---|---|---|---|
| $C22_{0,70}$ $C24_{0,28}$ $C20_{0,02}$ | T=+45,2°C, | δ=0,8, | ΔH=153 J/g |
| $C22_{0,68}$ $C24_{0,30}$ $C26_{0,02}$ | T=+45,7°C, | δ=0,8, | ΔH=153 J/g |
| $C22_{0,30}$ $C24_{0,68}$ $C20_{0,02}$ | T=+48,1°C, | δ=0,9, | ΔH=155 J/g |

| | | | |
|---|---|---|---|
| C220,28 C240,70 $C23_{0,02}$ | T=+48,4°C, | δ=0,9 | ΔH=156 J/g |

C23-C25 : $\varepsilon_n = 0,91$ ; ΔT = 6,1°C

| | | | |
|---|---|---|---|
| $C23_{0,100}$ $C25_{0,890}$ $C24_{0,010}$ | T = +53,0°C, | δ=0,9, | ΔH = 161 J/g |

C24-C26 : $\varepsilon_n = 0,92$ ; ΔT = 5,5°C

| | | | |
|---|---|---|---|
| $C24_{0,90}$ $C26_{0,08}$ $C20_{0,02}$ | T = +50,2°C, | δ=0,4, | ΔH=158 J/g |
| $C24_{0,49}$ $C26_{0,50}$ $C20_{0,01}$ | T = +52,2°C, | δ=0,9, | ΔH=158 J/g |
| $C24_{0,49}$ $C26_{0,50}$ $C22_{0,01}$ | T = +53,5°C, | δ=0,19, | ΔH=162 J/g |

Si n continue à augmenter, on peut obtenir facilement des alliages où quel que soit x, le δ est inférieur à 0,5 même pour les concentrations centrales. On donne deux exemples :

C30-C32 : $\varepsilon_n$ = 0,93 ; ΔT = 3,9°C
δ ≤ 0,5 pour tout x de $C30_{1-x}$ $C32_x$

exemple : $C30_{0,50}$ $C32_{0,50}$ T = +67,0°C, δ = 0,2, ΔH=160 J/g

C44-C46 : $\varepsilon_n$ = 0,95 ; ΔT = 1,6°C
δ < 0,5 pour tout x de $C44_{1-x}$ $C46_x$

exemple : $C44_{0,50}$ $C46_{0,50}$ T = +87,4°C,δ = 0,2, ΔH=220 J/g

2) ALCAL non consécutifs

1er cas type Cnp C(np + 4) et type Cni C(ni + 4)

On retrouve sensiblement la même évolution que pour les ALCAL consécutifs avec une augmentation de l'étendue des domaines de miscibilité et une amélioration de la qualité de la fenêtre thermique au fur-et-à mesure que n augmente et que ΔT diminue, ce dernier paramètre étant relativement déterminant.

Il faut avoir $\varepsilon_n$ ≥ 0,80 pour avoir une miscibilité en toute proportion. Il faut, de plus, que ΔT ≤ 10°C pour que tous les alliages aient δ ≤ 2 Il faut que $\varepsilon_n$ ≥ T 0,90 pour que tous les alliages aient leur δ ≤ 1.

Exemples de formulations :

C18-C22 : $\varepsilon_n$ = 0,78 ; ΔT = 16,2°C

| | | | |
|---|---|---|---|
| $C18_{(1-x)}$ $C22_x$ recevables pour | 0,05 < x ≤ 0,20 avec δ = 2<br>0,20 < x ≤ 0,40 avec δ = 4<br>0,90 ≤ x ≤ 1 avec δ = 4 | | |
| $C18_{0,870}$ $C22_{0,100}$ $C20_{0,030}$: | T = +27,8 | δ = 1,8, | ΔH = 164 J/g |
| $C18_{0,700}$ $C22_{0,280}$ $C20_{0,020}$: | T = +30,8 | δ = 3,9, | ΔH = 139 J/g |

C22-C26 : $\varepsilon_n$ = 0,82 ; ΔT = 12,0°C

| | | | |
|---|---|---|---|
| C22 $_{(1-x)}$ $C26_x$ recevables pour | 0 ≤ x ≤ 0,35 avec δ = 2<br>0,35 < x < 0,75 avec δ = 4<br>0,75 ≤ x ≤ 1 avec δ = 2 | | |
| $C22_{0,500}$ $C26_{0,490}$ $C24_{0,010}$: | T = +50,2°C, | δ = 2,6, | ΔH =157 J/g |

C28-C32 : $\varepsilon_n$ = 0,86 ; ΔT = 8,3°C

| | | | |
|---|---|---|---|
| C28(1-x)$C32_x$ recevables pour | 0 ≤ x ≤ 0,30 avec δ = 1<br>0,30 < x ≤ 0,90 avec δ = 2<br>0,90 < x ≤ 1 avec δ = 1 | | |
| $C28_{0,790}$ $C32_{0,200}$ $C30_{0,010}$: | T = +62,4°C, | δ = 0,4, | ΔH =160 J/g |

C46-C50 : $\varepsilon_n$ = 0,91 ; ΔT = 4,1°C

$\delta < 1$ quelle que soit la teneur
$C46_{0,100}C50_{0,890}C44_{0,010}$:T = +91,9°C $\delta$ = 0,6, $\Delta H$ = 215 J/g

2ème cas : type Cnp C(np + k) et type Cni C(ni + k) où k est un nombre pair supérieur à 4.

Pour obtenir des domaines de miscibilité assez étendus avec des 6 de bonne qualité, on utilise des np (ou ni) relativement élevés de façon à avoir simultanément
$\varepsilon_n > 0,80$ et $\Delta T < 10°C$

Exemples de binaires avec k = 6

C26-C32 : $\varepsilon_n$ = 0,77 ; $\Delta T$ = 13,3°C
La miscibilité est totale mais le fuseau est large du côté du C32. De telle sorte que l'on a :

| | | | |
|---|---|---|---|
| $C26_{(1-x)}\ C32_x$ recevables pour $0 \le x \le 0,10$ avec $\delta = 1$ | | | |
| | $0,10 < x \le 0,25$ avec $\delta = 2$<br>$0,25 < x \le 0,45$ avec $\delta = 4$<br>$0,90 \le x \le 1$ avec $\delta = 4$ | | |
| $C26_{0,800}\ C32_{0,190}\ C30_{0,010}$: | T = +57,3°C, | $\delta$ = 2,0, | $\Delta H$=160J/g |

C44-C50 : $\varepsilon_n$ = 0,86 ; $\Delta T$ = 5,7°C

| | | | |
|---|---|---|---|
| $C44_{(1-x)}\ C50_x$ recevables pour tout x | | | |
| | pour $0 \le x \le 0,32$ avec $\delta = 1$<br>$0,32 < x < 0,75$ avec $\delta = 2$<br>$0,75 \le x \le 1$ avec $\delta = 1$ | | |
| $C44_{0,395}C50_{0,585}C46_{0,005}C48_{0,015}$: | T=+89,2°C, | $\delta$=1,5, | $\Delta H$=225J/g |

II ALCAL binaires avec parité différente

A et B appartiennent l'un aux alcanes pairs et l'autre aux alcanes impairs ou vice versa.

1) ALCAL consécutifs

Ils sont de type Cnp/Cni avec i = p + 1 ou Cni/Cnp avec p = i + 1
Comme dans les cas précédents, on observe un effet favorable de l'allongement de la chaîne. Néanmoins doit être considéré ici l'effet de non isomorphisme qui caractérise les couples Cnp/Cni ou Cni/Cnp tant que les n sont inférieurs à 21. On distingue donc deux cas :

1er cas :

Au moins l'un des constituants possède un n < 21. Dans ce cas, la miscibilité ne saurait être totale mais, dès que les $\varepsilon_n$ sont supérieurs à 0,90, on observe, pour chaque binaire, 2 fuseaux partiels relativement étroits, se coupant en une zone exclue selon l'invention du point de vue 6, zone systématiquement décalée vers le Cn pair.

Exemples de binaires :

C12-C13 : $\varepsilon_n = 0,92$ ; $\Delta T = 4,1°C$
On a des alliages $C12_{(1-x)}\ C13_x$ recevables dès lors que
$x \geq 0,30$
$0,30 \leq x < 0,90$ avec $\delta = 4$
$0,90 \leq x \leq 1$ avec $\delta = 2$
avec notamment :
les formulations non dopées suivantes :

| | | | |
|---|---|---|---|
| $C12_{0,700}\ C13_{0,300}$ | T = -13,6°C, | $\delta$ = 2,6, | $\Delta$H = 146 J/g |
| $C12_{0,500}\ C13_{0,500}$ | T = -11,4°C, | $\delta$ = 2,2, | $\Delta$H = 146 J/g |

les formulations dopées suivantes :

| | | | |
|---|---|---|---|
| $C12_{0,690}C13_{0,300}C11_{0,010}$ | T = -13,5°C, | $\delta$ = 2,7, | $\Delta$H = 146J/g |
| $C12_{0,480}C13_{0,480}C11_{0,040}$ | T = -11,8°C, | $\delta$ = 2,2, | $\Delta$H = 146J/g |
| $C12_{0,200}C13_{0,780}C11_{0,020}$ | T = -8,0°C, | $\delta$ = 2,4, | $\Delta$H = 150J/g |

C13-C14 : $\varepsilon_n = 0,92$, $\Delta T = 11,4°C$
On a des alliages $C13_{(1-x)}\ C14_x$ dès lors que $x \leq 0,50$
$0 \leq x < 0,07$ avec $\delta = 1$
$0,07 \leq x \leq 0,50$ avec $\delta = 2$
avec notamment
comme formulations non dopées :

| | | | |
|---|---|---|---|
| $C13_{0,700}\ C14_{0,300}$ | T = -3,8°C, | $\delta$ = 1,2, | $\Delta$H = 153 J/g |
| $C13_{0,500}\ C14_{0,500}$ | T = -2,3°C, | $\delta$ = 1,7, | $\Delta$H = 152 J/g |

comme formulations dopées :

| | | | |
|---|---|---|---|
| $C13_{0,690}C14_{0,300}C15_{0,010}$ | T = -3,9°C, | $\delta$ = 1,4, | $\Delta$H = 152 J/g |
| $C13_{0,485}C14_{0,490}C15_{0,025}$ | T = -2,5°C, | $\delta$ = 1,8, | $\Delta$H = 152 J/g |

C14-C15 : $\varepsilon_n = 0,93$, $\Delta T = 4,1°C$
On a des alliages $C14_{(1-x)}\ C15_x$ lorsque $x > 0,20$
$0,20 < x < 0,70$ avec $\delta = 4$
$0,70 \leq x < 0,90$ avec $\delta = 2$
$0,90 \leq x \leq 1$ avec $\delta = 1$

exemples de formulations dopées :

| | | | |
|---|---|---|---|
| $C14_{0,400}C15_{0,570}C13_{0,030}$ | T = +5,2°C, | $\delta$ = 3,9, | $\Delta$H = 147 J/g |
| $C14_{0,260}C15_{0,700}C16_{0,040}$ | T = +7,7°C, | $\delta$ = 2,0, | $\Delta$H = 155 J/g |

C15-C16 : $\varepsilon_n = 0,93$, $\Delta T = 8,2°C$
On a des alliages $C15_{(1-x)}\ C16_x$ sauf si $0,70 < x < 0,90$
$0 \leq x \leq 0,25$ avec $\delta = 1$
$0,25 < x \leq 0,70$ avec $\delta = 2$
$0,90 \leq x \leq 1$ avec $\delta = 4$
exemples de formulations dopées

| | | | |
|---|---|---|---|
| $C15_{0,840}\ C16_{0,130}\ C14_{0,030}$ | T= +9,5°C, | $\delta$ = 1,0, | $\Delta$H = 153 J/g |

(suite)

| | |
|---|---|
| $C15_{0,840}$ $C16_{0,150}$ $C14_{0,010}$ | T= +10,5°C, $\delta$ = 0,8, $\Delta$H = 150 J/g |
| $C15_{0,690}$ $C16_{0,300}$ $C14_{0,010}$ | T= +11,2°C, $\delta$ = 1,3, $\Delta$H = 156 J/g |

<u>C16-C17</u> : $\varepsilon_n$ = 0,94, $\Delta$T = 3,8°C
On a des alliages $C16_{(1-x)}$ $C17_x$ si x > 0,10
0,10 < x ≤ 0,40 avec $\delta$ = 1
0,40 < x ≤ 0,90 avec $\delta$ = 2
0,90 < x ≤ 1 avec $\delta$ = 1
exemples de formulations dopées :

| | |
|---|---|
| $C16_{0,300}C17_{0,685}C15_{0,010}C18_{0,005}$ | T=19,8°C, $\delta$=1,5, $\Delta$H =160J/g |
| $C16_{0,100}C17_{0,885}C15_{0,005}C18_{0,010}$ | T=21,3°C, $\delta$=1,1, $\Delta$H =165J/g |

<u>C17-C18</u> : $\varepsilon_n$ = 0,94 ; $\Delta$T = 6,4°C
On a des alliages $C17_{(1-x)}$ $C18_x$ pour x < 0,75
0 ≤ x ≤ 0,50 avec $\delta$ = 1
0,50 < x < 0,75 avec $\delta$ = 2
exemple de formulation dopée :

| | |
|---|---|
| $C17_{0,300}C18_{0,690}C19_{0,010}$ | T = +25,7°C, $\delta$ = 1,2, $\Delta$H =159 J/g |

<u>C19-C20</u> : $\varepsilon_n$ = 0,95 ; $\Delta$T = 4,7°C
On a des alliages $C19_{(1-x)}C20_x$ avec $\delta$ < 1 quel que soit x sauf entre 0,90 < x < 1
exemple de formulation dopée :

| | |
|---|---|
| $C19_{0,300}C20_{0,680}C21_{0,020}$ | T = +35,2°C, $\delta$=0,9, $\Delta$H = 151 J/g |

<u>2ème cas</u> : tous les constituants ont un n > 21

Les phases avant fusion des alcanes pairs et impairs étant isomorphes, la miscibilité totale est possible et intervient effectivement d'autant que tous les $\varepsilon_n$ sont supérieurs ou égaux à 0,95.

<u>C21-C22</u> : $\varepsilon_n$ = 0,95 ; $\Delta$T = 3,9°C
Tous les alliages $C21_{(1-x)}$ $C22_x$ sont recevables avec $\delta$ < 1.
exemple de formulation dopée :

| | |
|---|---|
| $C21_{0,490}$ $C22_{0,490}$ $C23_{0,020}$ | T= +41,8°C, $\delta$ = 0,6, $\Delta$H = 150J/g |

<u>C22-C23</u> : $\varepsilon_n$ = 0,95 ; $\Delta$T = 3,2°C
Tous les alliages $C22_{(1-x)}$ $C23_x$ sont recevables avec $\delta$ < 1.
exemple de formulation dopée :

| | |
|---|---|
| $C22_{0,490}C23_{0,500}C24_{0,010}$ | T = +45,7°C, $\delta$ = 0,5, $\Delta$H = 155 J/g |

<u>C24-C25 :</u> $\varepsilon_n$ = 0,96 ; $\Delta$T = 2,8°C
exemple de formulation dopée :

| | |
|---|---|
| $C24_{0,500}C25_{0,490}C26_{0,010}$ | T=+51,7°C, $\delta$ = 0,4, $\Delta$H = 159 J/g |

<u>C35-C36</u> : $\varepsilon_n$ = 0,97 ; T = 1,1°C
exemple de formulation dopée :

| | |
|---|---|
| $C35_{0,890}C36_{0,090}C37_{0,020}$ | T=+75,2°C, δ = 0,4, ΔH = 160 J/g |

2) ALCAL non consécutifs

Il convient, si l'on veut de larges domaines de concentrations recevables, de choisir les constituants de telle sorte que leur $\varepsilon_n$ soit supérieur à 0,90.

Exemple 5 : **ALCAL ternaires :**

**a) étude de l'évolution.**

Les ternaires seront présentés de la façon suivante Cn1 - Cn2 - Cn3 avec n1 < n2 < n3 ce qui correspond également à l'ordre croissant des températures de fusion.
Pour chaque ternaire, on donnera les $\varepsilon_n$ pour les constituants pris deux à deux selon l'ordre suivant Cn1 - Cn2 ; Cn2 - Cn3 et Cn1 - Cn3. Cette troisième valeur sera toujours la plus faible compte tenu de la systématique adoptée.

1) Ternaires avec Cn tous consécutifs : Il s'agit de ternaires Cn-C(n + 1)-C(n + 2)

A titre d'exemple on citera :

| Cn1 Cn2 Cn3 | $\varepsilon_{1,2}$ | $\varepsilon_{2,3}$ | $\varepsilon_{1,3}$ |
|---|---|---|---|
| C14 - C15 - C16 | 0,93 | 0,93 | 0,86 |
| C15 - C16 - C17 | 0,93 | 0,94 | 0,87 |
| C20 - C21 - C22 | 0,95 | 0,95 | 0,90 |
| C22 - C23 - C24 | 0,95 | 0,96 | 0,91 |

L 'étendue des plages de concentrations recevables augmente au fur-et-à-mesure que les divers $\varepsilon_{1,2}$ ; $\varepsilon_{2,3}$ et $\varepsilon_{1,3}$ s'accroissent. Pour les systèmes C14 - C15 - C16 et C15 - C16 - C17 où les $\varepsilon_{1,3}$ < 0,90 les plages de qualité δ = 1 et δ = 2 sont peu étendues et il existe des plages où δ > 4. Si tous les $\varepsilon_n$ ≥ 0,90, toutes les formulations ont au moins le label δ ≤ 2 (cas C20 - C21 - C22 à l'exception d'un petit domaine perturbé par la transition solide - solide du C20) et même le label 6 ≤ 1 (cas C22-C23-C24).

2) Ternaires avec Cn non directement consécutifs

1er cas général : ternaires Cn-C(n + 2)-C(n + 4)

Ce sont par exemple les ternaires suivants (dont 3 cas sont détaillés par la suite).

| Cn1 - Cn2 - Cn3 | $\varepsilon_{1,2}$ | $\varepsilon_{2,3}$ | $\varepsilon_{1,3}$ |
|---|---|---|---|
| C18 - C20 - C22 | 0,89 | 0,90 | 0,78 |
| C20 - C22 - C24 | 0,90 | 0,91 | 0,80 |
| C21 - C23 - C25 | 0,90 | 0,91 | 0,81 |
| C22 - C24 - C26 | 0,91 | 0,92 | 0,82 |

On retrouve le même phénomène que précédemment : augmentation de l'étendue des plages de meilleur label avec augmentation des trois $\varepsilon_n$ concernés, le $\varepsilon_{1,3}$ étant le plus déterminant puisque mettant en jeu les deux Cn les plus éloignés en longueur de chaîne.

2ème cas général

Plus les écarts vont être grands entre les divers Cn impliqués et plus il faudra augmenter dans l'échelle des alcanes, classés selon leur n, pour avoir des plages importantes d'ALCAL possédant un bon δ. Deux exemples complets sont fournis ici :

| | | | |
|---|---|---|---|
| C20 - C22 - C26 | 0,90 | 0,82 | 0,70 |
| C44 - C46 - C50 | 0,95 | 0,91 | 0,86 |

Si pour le premier, il y a de larges plages de formulations à label $\delta > 6$, pour le second, par contre, la plupart des formulations ont un label $\delta \leq 1$ et les autres ont un $1 < \delta \leq 2$.

**b) : Exemples**

**1. Etude des ALCAL ternaires xC14-yC15-zC16**

Le diagramme donnant les labels des différents alliages est rapporté sur la figure 1. Sur ce diagramme, on a distingué 8 zones (surfaces numérotées de $S_1$ à $S_8$).

Une zone où $\delta \leq 1$ : $S_1$

. Définition de la zone $S_1$

( $18x - 2y + 3z \leq 0$

( $y \geq 0,75$

( $x + y + z = 1$

avec notamment les exemples suivants :

| | | | |
|---|---|---|---|
| x = 0,03 | y = 0,84 | z = 0,13 | $T_\delta = +9,5_{1,0}$ |
| x = 0,01 | y = 0,84 | z = 0,15 | $T_\delta = +10,5_{0,8}$ |

Deux zones où $1 < \delta \leq 2$ : $S_2$ et $S_3$

. Définition de la zone $S_2$ = S n'incluant pas $S_1$

où S ( $49x - 21y + 9z \leq 0$

( $x + y + z = 1$

avec notamment les exemples suivants :

| | | | |
|---|---|---|---|
| x = 0,01 | y = 0,49 | z = 0,50 | $T_\delta = +12,3_{1,7}$ |
| x = 0,01 | y = 0,69 | z = 0,30 | $T_\delta = +11,2_{1,3}$ |
| x = 0,07 | y = 0,56 | z = 0,37 | $T_\delta = +10,7_{2,0}$ |
| x = 0,26 | y = 0,67 | z = 0,07 | $T_\delta = +7,7_{2,0}$ |

. Définition de la zone $S_3$

( $x \geq 0,7$

( $3y - 5z \leq 0$

( $x - 4y - z \geq 0$

( $x + y + z = 1$

avec notamment les exemples suivants :

| | | | |
|---|---|---|---|
| x = 0,73 | y = 0,14 | z = 0,13 | $T_\delta = +3,6_{2,0}$ |
| x = 0,89 | y = 0,01 | z = 0,10 | $T_\delta = +3,2_{1,9}$ |
| x = 0,79 | y = 0,01 | z = 0,20 | $T_\delta = +2,9_{2,0}$ |

Deux zones où $2 < \delta \leq 4$ : S4 et S5

. Définition de S4

( $4x + 29y - z \leq 0$

( $x + y + z = 1$

avec notamment l'exemple suivant :

| | | | |
|---|---|---|---|
| x = 0,10 | y = 0,01 | z = 0,89 | $T_\delta$ = +16,0$_{3,2}$ |

. Définition de $S_5$
triangle total excluant : $S_1$ $S_2$ $S_3$ $S_4$ $S_6$ $S_7$ et $S_8$
avec notamment les exemples suivants :

| | | | |
|---|---|---|---|
| x = 0,14 | y = 0,23 | z = 0,63 | $T_\delta$ = +11,9$_{3,1}$ |
| x = 0,21 | y = 0,56 | z = 0,23 | $T_\delta$ = +8,5$_{2,3}$ |
| x = 0,24 | y = 0,33 | z = 0,43 | $T_\delta$ = +9,5$_{3,9}$ |
| x = 0,43 | y = 0,33 | z = 0,24 | $T_\delta$ = +6,5$_{2,6}$ |
| x = 0,57 | y = 0,17 | z = 0,26 | $T_\delta$ = +5,3$_{2,6}$ |
| x = 0,64 | y = 0,01 | z = 0,35 | $T_\delta$ = +4,7$_{2,8}$ |

<u>Deux zones où $4 < \delta \leq 6$ : $S_6$ et $S_7$</u>

. Définition de $S_6$
( $x \geq 0,2$
( $y \leq 0,3$
( $19x + 9y - 21z \leq 0$
( $x + y + z = 1$
avec notamment les exemples suivants :

| | | | |
|---|---|---|---|
| x = 0,33 | y = 0,07 | z = 0,60 | $T_\delta$ = +9,9$_{4,4}$ |
| x = 0,41 | y = 0,01 | z = 0,58 | $T_\delta$ = +8,8$_{4,9}$ |
| x = 0,32 | y = 0,24 | z = 0,44 | $T_\delta$ = +8,8$_{4,4}$ |

. Définition de S7
( $x - 4y - z \geq 0$
( $3y - 5z > 0$
( $x + y + z = 1$

<u>Une zone où $\delta > 6$ : $S_8$</u> (*).
Définition de $S_8$
( $x \leq 0,2$
( $z \geq 0,7$
( $4x + 29y - z > 0$
( $x + y + z = 1$
avec notamment l'exemple suivant :

| | | | |
|---|---|---|---|
| x = 0,17 | y = 0,07 | z = 0,76 | $T_\delta$ = +13,8$_{7,4}$ |

**2. <u>Etude des ALCAL ternaires xC15-yC16-zC17</u>**

Le diagramme donnant les labels des différents alliages est représenté sur la figure 2.
On distingue 5 zones (surfaces renumérotées de $S_1$ à S5).
<u>Une zone où $\delta \leq 1$ : $S_1$</u>

. Définition de $S_1$
( $3x + 9y - 17z \geq 0$
( $x + y + z = 1$
avec notamment les exemples suivants :

(*) comparatifs

| | | | |
|---|---|---|---|
| x = 0,83 | y = 0,15 | z = 0,02 | $T_\delta = +10{,}6_{0,8}$ |
| x = 0,87 | y = 0,07 | z = 0,06 | $T_\delta = +10{,}3_{1,0}$ |

Une zone où $1 < \delta \leq 2$ : $S_2$

. Définition de $S_2$
triangle total excluant $S_1$ $S_3$ $S_4$ $S_5$
avec notamment les exemples suivants :

| | | | |
|---|---|---|---|
| x = 0,77 | y = 0,17 | z = 0,06 | $T_\delta = +10{,}8_{1,3}$ |
| x = 0,77 | y = 0,07 | z = 0,16 | $T_\delta = +10{,}9_{1,4}$ |
| x = 0,67 | y = 0,30 | z = 0,03 | $T_\delta = +11{,}4_{1,4}$ |
| x = 0,50 | y = 0,48 | z = 0,02 | $T_\delta = +12{,}4_{1,7}$ |
| x = 0,36 | y = 0,47 | z = 0,17 | $T_\delta = +13{,}5_{2,0}$ |
| x = 0,27 | y = 0,66 | z = 0,07 | $T_\delta = +14{,}9_{2,0}$ |
| x = 0,70 | y = 0,03 | z = 0,27 | $T_\delta = +11{,}4_{1,4}$ |
| x = 0,06 | y = 0,67 | z = 0,27 | $T_\delta = +16{,}5_{1,9}$ |
| x = 0,03 | y = 0,27 | z = 0,70 | $T_\delta = +19{,}5_{1,6}$ |

Une zone où $2 < \delta \leq 4$ : $S_3$

. Définition de la zone $S_3$ : S n'incluant pas $S_5$

$$S \left( \begin{array}{l} 133x - 17y - 7z \geq 0 \\ 6x - 4y + 11z \geq 0 \\ 2x - 3z \leq 0 \\ x \leq 0,3 \\ x + y + z = 1 \end{array} \right.$$

avec notamment les exemples suivants :

| | | | |
|---|---|---|---|
| x = 0,44 | y = 0,23 | z = 0,33 | $T_\delta = +14{,}1_{2,6}$ |
| x = 0,27 | y = 0,47 | z = 0,26 | $T_\delta = +14{,}8_{2,3}$ |
| x = 0,27 | y = 0,07 | z = 0,66 | $T_\delta = +17{,}1_{3,9}$ |
| x = 0,06 | y = 0,07 | z = 0,87 | $T_\delta = +20{,}4_{2,5}$ |

Deux zones où $\delta > 4$ : $S_4$ et $S_5$

. Définition de $S_4$
( $0,2 \leq x \leq 0,45$
( $0 \leq y \leq 0,05$
( $0,5 \leq z \leq 0,8$
( $x + y + z = 1$
avec notamment l'exemple suivant :

| | | | |
|---|---|---|---|
| x = 0,40 | y = 0,02 | z = 0,58 | $T_\delta = +15{,}8_{4,3}$ |

. Définition de $S_5$
( $7x - 3y + 17z \leq 0$
( $x + y + z = 1$

avec notamment l'exemple suivant :

| | | | |
|---|---|---|---|
| x = 0,10 | y = 0,88 | z = 0,02 | $T_\delta = +17{,}0_{4,2}$ |

**3 : Etude des ALCAL ternaires xC20-yC21-zC22**

La figure 3 représente le diagramme donnant les labels des différents alliages. On distingue 3 zones (surfaces numérotées de $S_1$ à $S_3$)

Une zone où $\delta \leq 1$ : $S_1$

. Définition de la zone $S_1$
triangle total excluant $S_2$ et $S_3$
avec notamment les exemples suivants :

| | | | |
|---|---|---|---|
| x = 0,09 | y = 0,01 | z = 0,90 | $T_\delta = +42{,}8_{0,9}$ |
| x = 0,50 | y = 0,47 | z = 0,03 | $T_\delta = +38{,}2_{0,6}$ |
| x = 0,80 | y = 0,03 | z = 0,17 | $T_\delta = +37{,}5_{0,8}$ |
| x = 0,03 | y = 0,47 | z = 0,50 | $T_\delta = +41{,}6_{0,6}$ |

. Une zone où $1 < \delta \leq 2$ : $S_2$

. Définition de la zone $S_2$
( $3x - y - z \geq 0$
( $2x - 3y + 2z \geq 0$
( $6x + y - 9z \leq 0$
( $x + y + z = 1$

| | | | |
|---|---|---|---|
| x = 0,30 | y = 0,01 | z = 0,69 | $T_\delta = +41{,}2_{1,4}$ |
| x = 0,48 | y = 0,03 | z = 0,49 | $T_\delta = +39{,}7_{1,5}$ |
| x = 0,33 | y = 0,34 | z = 0,33 | $T_\delta = +39{,}5_{1,3}$ |

Une zone où $\delta > 4$ : $S_3$

. Définition de la zone $S_3$
( $x \geq 0{,}95$
( $x + y + z = 1$

**4 : Etude des ALCAL ternaires xC22-yC23-zC24**

Le diagramme des labels des différents alliages est représenté sur la figure 4 est caractérisé par l'existence d'une seule zone appelée S où $\delta \leq 1$

. Définition de la zone S :
tout le ternaire, soit $x + y + z = 1$
avec notamment les exemples suivants :

| | | | |
|---|---|---|---|
| x = 0,05 | y = 0,02 | z = 0,93 | $T_\delta = +50{,}2_{0,4}$ |
| x = 0,28 | y = 0,02 | z = 0,70 | $T_\delta = +48{,}4_{0,9}$ |
| x = 0,33 | y = 0,34 | z = 0,33 | $T_\delta = +47{,}1_{0,7}$ |
| x = 0,70 | y = 0,02 | z = 0,28 | $T_\delta = 45{,}7_{0,9}$ |
| x = 0,48 | y = 0,50 | z = 0,02 | $T_\delta = 45{,}8_{0,6}$ |
| x = 0,03 | y = 0,47 | z = 0,50 | $T_\delta = 48{,}9_{0,5}$ |

**5 : Etude des ALCAL ternaires xC18-yC20-zC22**

Le diagramme des labels des différents alliages est donné sur la figure 5. On distingue 7 zones (surfaces numérotées de $S_1$ à $S_7$).

Une zone où $\delta \leq 1$ : $S_1$

. Définition de la zone $S_1$ : S n'incluant pas $S_7$

$$S \begin{cases} 18x - 2y + 3z \leq 0 \\ x + y + z = 1 \end{cases}$$

avec notamment les exemples suivants :

| | | | |
|---|---|---|---|
| x = 0,06 | y = 0,88 | z = 0,06 | $T_\delta = +36,1_{1,0}$ |
| x = 0,03 | y = 0,77 | z = 0,20 | $T_\delta = +37,5_{0,9}$ |

Une zone où $1 < \delta \leq 2$ : $S_2$

. Définition de la zone $S_2$ :
triangle total excluant $S_1$, $S_3$, $S_4$, $S_5$, $S_6$ et $S_7$
avec notamment les exemples suivants :

| | | | |
|---|---|---|---|
| x = 0,78 | y = 0,20 | z = 0,02 | $T_\delta = +28,5_{2,0}$ |
| x = 0,53 | y = 0,44 | z = 0,03 | $T_\delta = +30,9_{2,0}$ |
| x = 0,19 | y = 0,80 | z = 0,01 | $T_\delta = +33,9_{2,0}$ |
| x = 0,63 | y = 0,24 | z = 0,13 | $T_\delta = +30,0_{1,9}$ |
| x = 0,06 | y = 0,56 | z = 0,38 | $T_\delta = +37,8_{1,3}$ |
| x = 0,40 | y = 0,58 | z = 0,02 | $T_\delta = +40,1_{1,5}$ |
| x = 0,18 | y = 0,80 | z = 0,02 | $T_\delta = +41,8_{1,1}$ |
| x = 0,87 | y = 0,10 | z = 0,03 | $T_\delta = +27,8_{1,8}$ |

Une zone où $2 < \delta \leq 4$ : $S_3$

. Définition de la zone $S_3$ : S excluant $S_4$ et $S_5$

$$S \begin{cases} 4x - y \geq 0 \\ z \geq 0,2 \\ x + y + z = 1 \end{cases}$$

avec notamment les exemples suivants :

| | | | |
|---|---|---|---|
| x = 0,23 | y = 0,54 | z = 0,23 | $T_\delta = +35,3_{2,7}$ |
| x = 0,33 | y = 0,34 | z = 0,33 | $T_\delta = +35,7_{3,7}$ |
| x = 0,13 | y = 0,33 | z = 0,54 | $T_\delta = +38,6_{2,6}$ |
| x = 0,70 | y = 0,28 | z = 0,02 | $T_\delta = +30,8_{3,9}$ |

Une zone où $4 < \delta \leq 6$ : $S_4$

. Définition de la zone $S_4$

( $2x + 7y - 3z > 0$
( $y \leq 0,3$
( $z \geq 0,4$
( $9x - 6y - z \geq 0$
( $x + y + z = 1$

avec notamment l'exemple suivant :

| | | | |
|---|---|---|---|
| x = 0,43 | y = 0,14 | z = 0,43 | $T_\delta = +35,8_{5,1}$ |

Trois zones où $\delta > 6$ : $S_5$, $S_6$, et $S_7$ (*).

Définition de $S_5$ :

( $2x + 7y - 3z < 0$
( $9x - 6y - z \geq 0$
( $x + y + z = 1$

avec notamment les exemples suivants :

| | | | |
|---|---|---|---|
| x = 0,48 | y = 0,02 | z = 0,50 | $T_\delta = +36,3_{6,5}$ |
| x = 0,27 | y = 0,06 | z = 0,67 | $T_\delta = +39,2_{7,4}$ |

. Définition de $S_6$

( $x \geq 0,95$
( $x + y + z = 1$

. Définition de $S_7$

( $9x - y + 19z \leq 0$
($x + y + z = 1$

**6 : Etude des ALCAL ternaires xC20-yC22-zC24**

Sur le diagramme donnant les labels des différents alliages, représenté sur la figure 6, on distingue 5 zones (surfaces numérotées de $S_1$ à $5_5$)

Deux zones où $\delta \leq 1$ : $S_1$ et $S_2$

. Définition de $S_1$ :

( $5x - y \leq 0$
( $x + y + z = 1$

avec notamment les exemples suivants :

| | | | |
|---|---|---|---|
| x = 0,02 | y = 0,30 | z = 0,68 | $T_\delta = +48,1_{0,9}$ |
| x = 0,02 | y = 0,70 | z = 0,28 | $T_\delta = +45,2_{0,8}$ |
| x = 0,07 | y = 0,76 | z = 0,17 | $T_\delta = +44,7_{0,9}$ |

. Définition de $S_2$ :

( $- 2x + 3y + 18z \leq 0$
( $x + y + z = 1$

avec notamment les exemples suivants :

| | | | |
|---|---|---|---|
| x = 0,78 | y = 0,20 | z = 0,02 | $T_\delta = +38,0_{0,8}$ |

Une zone où $1 \leq \delta < 2$ : $S_3$

. Définition de $S_3$

triangle total excluant $S_1$, $S_2$, $S_4$ et $S_5$

avec notamment les exemples suivants :

(*) comparatifs

| | | | |
|---|---|---|---|
| x = 0,05 | y = 0,02 | z = 0,93 | $T_\delta$ = +50,5$_{1,1}$ |
| x = 0,17 | y = 0,66 | z = 0,17 | $T_\delta$ = +43,3$_{1,2}$ |
| x = 0,37 | y = 0,46 | z = 0,17 | $T_\delta$ = +42,0$_{1,9}$ |
| x = 0,38 | y = 0,60 | z = 0,02 | $T_\delta$ = +40,6$_{1,4}$ |
| x = 0,50 | y = 0,48 | z = 0,02 | $T_\delta$ = +40,0$_{1,5}$ |
| x = 0,66 | y = 0,17 | z = 0,17 | $T_\delta$ = +39,0$_{1,6}$ |
| x = 0,87 | y = 0,02 | z = 0,11 | $T_\delta$ = +38,6$_{1,2}$ |

Une zone où $2 < \delta \leq 4$ : $S_4$

. Définition de $S_4$ :

( $-36x + 9y + 4z \geq 0$
( $y \leq 0,4$
( $z \geq 0,2$
( $x + y + z = 1$

avec notamment les exemples suivants :

| | | | |
|---|---|---|---|
| x = 0,23 | y = 0,03 | z = 0,74 | $T_\delta$ = +47,0$_{3,0}$ |
| x = 0,17 | y = 0,37 | z = 0,46 | $T_\delta$ = +45,6$_{2,2}$ |
| x = 0,47 | y = 0,06 | z = 0,47 | $T_\delta$ = +43,4$_{3,7}$ |
| x = 0,34 | y = 0,33 | z = 0,33 | $T_\delta$ = +43,4$_{2,8}$ |
| x = 0,40 | y = 0,20 | z = 0,40 | $T_\delta$ = +43,3$_{3,3}$ |
| x = 0,66 | y = 0,07 | z = 0,27 | $T_\delta$ = +39,9$_{2,3}$ |

Une zone où $\delta > 4$ : $S_5$

. Définition de $S_5$

( $x - 19y - 9z \geq 0$
( $x + y + z = 1$

**7 : Etude des ALCAL ternaires xC22-yC24-zC26**

Le diagramme donnant les labels des différents alliages est représenté sur la figure 7. On distingue trois zones (surfaces numérotées de $S_1$ à $S_3$)

Une zone où $\delta \leq 1$ : $S_1$

. Définition de $S_1$ :

( $17x - 3y \leq 0$
( $y - 2z \geq 0$
( $2x - 3y + 2z \leq 0$
( $56x - 34y + 6z \leq 0$
( $x + y + z = 1$

avec notamment les exemples suivants :

| | | | |
|---|---|---|---|
| x = 0,01 | y = 0,49 | z = 0,50 | $T_\delta$ = +53,5$_{0,9}$ |
| x = 0,03 | y = 0,95 | z = 0,02 | $T_\delta$ = +50,5$_{0,5}$ |
| x = 0,17 | y = 0,66 | z = 0,17 | $T_\delta$ = +50,3$_{0,8}$ |
| x = 0,30 | y = 0,68 | z = 0,02 | $T_\delta$ = +48,6$_{0,9}$ |
| x = 0,46 | y = 0,37 | z = 0,17 | $T_\delta$ = +47,7$_{1,1}$ |
| x = 0,68 | y = 0,30 | z = 0,02 | $T_\delta$ = +45,7$_{0,8}$ |

Une zone où $1 < \delta \leq 2$ : $S_2$

. Définition de la zone $S_2$ :
triangle total excluant $S_1$ et $S_3$
avec notamment les exemples suivants :

| | | | |
|---|---|---|---|
| x = 0,10 | y = 0,18 | z = 0,72 | $T_\delta = +54,0_{1,9}$ |
| x = 0,66 | y = 0,07 | z = 0,27 | $T_\delta = +47,2_{1,6}$ |
| x = 0,34 | y = 0,33 | z = 0,33 | $T_\delta = +44,3_{1,4}$ |

Une zone où 2 < 6 ≤ 4 : $S_3$

. Définition de $S_3$ :
( 12x + y - 4z ≥ 0
( 7x - 18y + 2z ≥ 0
( x + 2y - 2z ≤ 0
( x + y + z = 1
avec notamment les exemples suivants :

| | | | |
|---|---|---|---|
| x = 0,27 | y = 0,07 | z = 0,66 | $T_\delta = +52,2_{2,4}$ |
| x = 0,50 | y = 0,01 | z = 0,49 | $T_\delta = +50,2_{2,6}$ |
| x = 0,40 | y = 0,20 | z = 0,40 | $T_\delta = +49,7_{2,1}$ |
| x = 0,47 | y = 0,06 | z = 0,47 | $T_\delta = +49,8_{2,4}$ |

8 : Etude des ALCAL ternaires xC20-yC22-zC26

Le diagramme donnant les labels des différents alliages est représenté sur la figure 8. On distingue sept zones (surfaces numérotées de $S_1$ à $S_7$).

Deux zones où δ ≤ 1 : $S_1$ et $S_2$

. Définition de la zone $S_1$
( 7x - 3y + 27z ≤ 0
( x + y + z = 1
avec notamment les exemples suivants :

| | | | |
|---|---|---|---|
| x = 0,20 | y = 0,78 | z = 0,02 | $T_\delta = +42,2_{0,9}$ |
| x = 0,07 | y = 0,90 | z = 0,03 | $T_\delta = +43,3_{1,0}$ |

. Définition de la zone $S_2$
( - 6x + 3y + 17z ≤ 0
( - 3x + 57y + 17z > 0
( x + y + z = 1
avec notamment les exemples suivants :

| | | | |
|---|---|---|---|
| x = 0,78 | y = 0,20 | z = 0,02 | $T_\delta = +37,8_{0,8}$ |
| x = 0,67 | y = 0,30 | z = 0,03 | $T_\delta = +38,6_{0,9}$ |

Deux zones où 1 < δ ≤ 2 : $S_3$ et $S_4$

. Définition de $S_3$
( 119x + 13y - 7z ≤ 0
( x + y + z = 1

. Définition de $S_4$ : S excluant $S_1$, $S_2$ et $S_7$

$$S \begin{cases} 6x + y - 24z \geq 0 \\ 14x - 21y + 39z \leq 0 \\ x + y + z = 1 \end{cases}$$

avec notamment les exemples suivants :

| | | | |
|---|---|---|---|
| x = 0,63 | y = 0,23 | z = 0,14 | $T_\delta = +39{,}5_{1,8}$ |
| x = 0,58 | y = 0,40 | z = 0,02 | $T_\delta = +39{,}1_{1,1}$ |
| x = 0,47 | y = 0,50 | z = 0,03 | $T_\delta = +40{,}4_{1,5}$ |
| x = 0,30 | y = 0,67 | z = 0,03 | $T_\delta = +41{,}8_{1,4}$ |
| x = 0,13 | y = 0,74 | z = 0,13 | $T_\delta = +43{,}8_{1,5}$ |

Une zone où $2 < \delta \leq 4$ : $S_5$ = S n'incluant pas $S_6$

. Définition de $S_5$ : S excluant $S_6$

$$S \begin{cases} 6x + y - 24z > 0 \\ 14x - 21y + 39z > 0 \\ 119x + 13y - 7z < 0 \\ x + y + z = 1 \end{cases}$$

avec notamment les exemples suivants :

| | | | |
|---|---|---|---|
| x = 0,03 | y = 0,47 | z = 0,50 | $T_\delta = +50{,}0_{3,5}$ |
| x = 0,37 | y = 0,46 | z = 0,17 | $T_\delta = +42{,}6_{2,6}$ |
| x = 0,77 | y = 0,03 | z = 0,20 | $T_\delta = +38{,}8_{2,1}$ |
| x = 0,10 | y = 0,02 | z = 0,88 | $T_\delta = +54{,}5_{3,7}$ |

Deux zones où $\delta \geq 6$ : $S_6$ et $S_7$ (*).

Définition de $S_6$

$$\begin{cases} x \geq 0{,}15 \\ y \leq 0{,}45 \\ z \geq 0{,}25 \\ x + y + z = 1 \end{cases}$$

avec notamment l'exemple suivant :

| | | | |
|---|---|---|---|
| x = 0,33 | y = 0,34 | z = 0,33 | $T_\delta = +46{,}6_{6,0}$ |

. Définition de $S_7$

$$\begin{cases} -3x + 57y + 17z \leq 0 \\ x + y + z = 1 \end{cases}$$

**9 : Etude des ALCAL ternaires xC44-yC46-zC50**

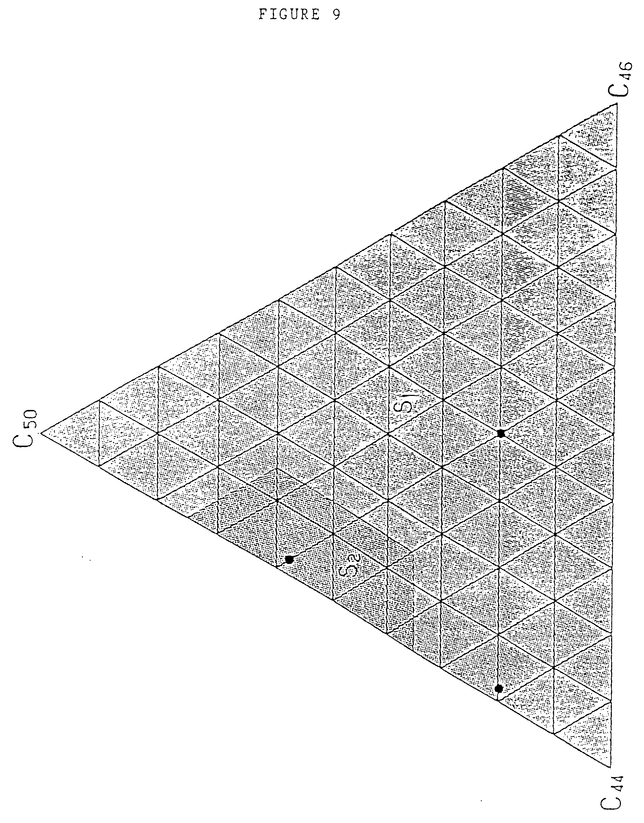

Sur le diagramme donnant les labels des différents alliages, représenté sur la figure 9, on distingue deux zones (surfaces numérotées de $S_1$ à $S_2$)

Une zone où $\delta \leq 1$ : $S_1$

(*) comparatifs

. Définition de $S_1$
Triangle total n'incluant pas $S_2$
avec notamment les exemples suivants :

| | | | |
|---|---|---|---|
| x = 0,78 | y = 0,02 | z = 0,20 | $T_\delta$ = +86,$7_{0,7}$ |
| x = 0,40 | y = 0,40 | z = 0,20 | Tδ = +87,$2_{0,5}$ |

Une zone où $1 < \delta \leq 2$ : $S_2$

. Définition de $S_2$ :
( $x \geq 0,25$
( $y \leq 0,15$
( $z \geq 0,35$
( $x + y + z = 1$
avec notamment l'exemple suivant :

| | | | |
|---|---|---|---|
| x = 0,40 | y = 0,02 | z = 0,58 | $T_\delta$ = +89,$2_{1,5}$ |

Exemple 6 : ALCAL quaternaires et plus

Les mêmes considérations sur les divers $\varepsilon_n$ des constituants pris deux à deux permettent l'élaboration d'ALCAL multicomposants recevables quant à leur label δ.

Exemples de formulations :

| | | | |
|---|---|---|---|
| $C14_{0,330}C15_{0,390}C16_{0,140}C18_{0,140}$ | T=+7,5°C | δ=3,4, | ΔH = 127J/g |
| $C14_{0,290}C15_{0,350}C16_{0,130}C17_{0,230}$ | T=+8,6°C | δ=4,0, | ΔH = 146J/g |
| $C44_{0,350}C46_{0,350}C48_{0,100}C50_{0,200}$ | T=+86,9°C | δ=0,6, | ΔH = 220J/g |
| $C14_{0,300}C15_{0,360}C16_{0,140}C17_{0,060}C18_{0,140}$ | T=+7,6°C, | δ = 3,5, | ΔH= 128 J/g |

Exemple 7 : Alliages moléculaires formés de chaînes monoacide-monoacide : On rapporte les résultats obtenus avec la formulation :

$[CH_3(CH_2)_{18}COOH]_{0,52}$ $[CH_3\ (CH_2)_{20}\ COOH]_{0,48}$ : T = +69,7°C, δ = 0,9, ΔH = 204 J/g

Exemple 8 : Alliages moléculaires formés de chaînes alcane-diacide :

avec la formulation suivante :

$[C_{22}\ H_{46}]_{0,80}$ $[COOH\ (CH_2)_{20}\ COOH]_{0,20}$, on obtient : T = +44,3°C, δ = 1,7, ΔH = 188 J/g

Exemple 9 : Application à la fabrication des plaques destinées aux poissonniers sur les marchés.

On recouvre un étal de plaques contenant des ALCAL de label $\delta \leq 4$. Par rapport à la glace couramment utilisée, ces plaques présentent l'avantage de pouvoir être régénérées en leur faisant subir un refroidissement. Le cas échéant, elles peuvent être utilisées sous une faible épaisseur de glace laquelle se conservera mieux si la présentation traditionnelle est souhaitée.

Exemple 10 : Application à la conservation au froid d'un aliment transporté :

Soit un aliment (solide ou liquide) que l'on veut pouvoir conserver dans son emballage dans un réfrigérateur classique pour le transporter tel quel jusqu'à un lieu donné (par exemple lieu de travail ou de pique-nique), pour le consommer quelques heures plus tard sans autre moyen de protection et sans que sa température ne dépasse 13°C (par exemple). On choisira avantageusement l'un des ALCAL figurant dans la liste de formulations ci-dessous. Le

choix est largement ouvert ; il s'effectuera en fonction des . paramètres à privilégier, notamment des contraintes économiques et de la disponibilité des produits de base :

| | T(°C) | δ(°C) | ΔHJ.g-1 |
|---|---|---|---|
| $C14_{0,214}C15_{0,561}C16_{0,225}$ | +8,5 | 2,3 | 152 |
| $C14_{0,290}C15_{0,350}C16_{0,130}C17_{0,220}C18_{0,010}$ | +8,6 | 4,1 | 146 |
| $C14_{0,320}C15_{0,240}C16_{0,440}$ | +8,8 | 4,4 | 151 |
| $C14_{0,400}C16_{0,600}$ | +9,0 | 4,9 | 148 |
| $C14_{0,107}C15_{0,592}C16_{0,220}C17_{0,059}C18_{0,022}$ | +9,4 | 2,3 | 145 |
| $C14_{0,030}C15_{0,840}C16_{0,130}$ | +9,5 | 1,0 | 153 |
| $C14_{0,240}C15_{3,330}C16_{0,430}$ | +9,5 | 3,9 | 151 |
| $C14_{0,330}C15_{0,070}C16_{0,600}$ | +9,9 | 4,4 | 151 |
| $C15_{0,870}C16_{0,070}C17_{0,060}$ | +10,3 | 1,0 | 158 |
| $C14_{0,010}C15_{0,840}C16_{0,150}$ | +10,5 | 0,8 | 150 |
| $C14_{0,030}C15_{0,640}C16_{0,330}$ | +10,7 | 2,0 | 152 |
| $C14_{0,066}C15_{0,505}C16_{0,369}$ | +10,7 | 2,1 | 158 |
| $C15_{0,770}C16_{0,170}C17_{0,060}$ | +10,8 | 1,3 | 157 |
| $C15_{0,770}C16_{0,070}C17_{0,160}$ | +10,9 | 1,4 | 154 |
| $C15_{0,700}C16_{0,300}$ | +11,2 | 1,3 | 156 |
| $C15_{0,660}C16_{0,250}C17_{0,070}C18_{0,030}$ | +11,2 | 1,9 | 152 |
| $C15_{0,700}C17_{0,300}$ | +11,3 | 1,3 | 146 |
| $C15_{0,685}C17_{0,300}C16_{0,015}$ | +11,3 | 1,3 | 146 |
| $C15_{0,670}C16_{0,300}C17_{0,030}$ | +11,4 | 1,4 | 155 |
| $C14_{0,140}C15_{0,230}C16_{0,630}$ | +11,9 | 3,1 | 147 |

A titre d'exemple, si l'on opte pour le premier alliage de cette liste, on disposera d'un MCPAM stockant à 95 % d'efficacité entre +6,2°C et +8,5°C. Il est évident que d'autres formulations convenables pourront être aisément mises au point par l'homme de l'art.

Il suffit de constituer un emballage à double paroi (enveloppant intégralement l'aliment) entre lesquelles sera placé l'ALCAL ; la couche d'ALCAL à employer sera fonction du temps de protection souhaité. L'ensemble étant placé au réfrigérateur, l'ALCAL va se solidifier (puisque la température y est inférieure à son Tsol), le tout va prendre la température de l'endroit du réfrigérateur où il est placé. Lors du transport et de la conservation à température ambiante de l'ensemble, l'ALCAL va faire barrière à l'arrivée des calories sur l'aliment proprement dit. En effet, les calories arrivant de l'extérieur vont, dans un premier temps, être absorbées par l'ALCAL pour élever sa température jusqu'à $T_{sol}$. Ensuite toutes les calories seront prises par l'ALCAL pour sa fusion et c'est l'énergie ΔH.m qui sera ainsi bloquée par le MCPAM (si m est sa masse). Ce n'est que lorsque tout l'ALCAL sera fondu que l'ensemble pourra voir sa température augmenter au-dessus de Tliq.

<u>Exemple 12</u> : Application aux enveloppements rafraîchissants et aux couvertures chauffantes.

Une couche d'ALCAL est incorporée dans l'enveloppement ou dans la couverture. Dans le premier cas, cet ALCAL. est choisi tel que sa température d'efficacité soit de l'ordre de +35°C. Un séjour en chambre fraîche suffit à donner ses propriétés au système, c'est-à-dire à rendre l'ALCAL solide. Le malade restera donc au contact d'une surface à +35°C, aussi longtemps que les calories qu'il dégage n'auront pas été suffisantes pour faire fondre l'intégralité du MCPAM.

Dans le second cas, on peut envisager l'emploi d'une résistance chauffante pour effectuer le stockage d'énergie, c'est-à-dire pour faire fondre un ALCAL fonctionnant à +38°C par exemple avec δ ≤ 2 ; l'utilisation se fait ensuite sans les risques habituels des couvertures chauffantes puisqu'à prise débranchée. Le maintien entre +38°C et +(38 - δ)°C persistera tant que le MCPAM n'aura pas achevé sa transition.

<u>Exemple 13</u> : Matelas chauffant pour tables d'opération et tables de soins.

Certaines opérations, de longue durée, nécessitent une aide au malade sous la forme d'un effort thermique pour compenser leur hypothermie. Le principe de fonctionnement de la couverture chauffante (ALCAL à T = +38°C et δ ≤ 2, avec résistance incorporée) pourra être repris en lui associant un système annexe à faible voltage (batterie par

exemple) associé à un contacteur cyclique pour entretenir l'ALCAL à l'équilibre solide-liquide durant une opération de longue durée ou lors du transport d'un malade. Dans cet exemple des MCPAM peuvent être associés à d'autres matériaux comme des matériaux fibreux ou expansés pour des raisons de confort. Des couches thermiques isolantes sur les faces externes (non en contact avec le patient) prolongeront l'autonomie de fonctionnement.

Exemple 14 : Appareil à fondue.

On réalise un système à résistance incorporée et fonctionnant à prise débranchée avec un MCPAM (à T supérieur à +100°C et avec un $\delta \leq 4$) placé dans une double paroi. L'appareil réalisé assure ainsi une haute sécurité lors de son fonctionnement. Les MCPAM pourront être choisis avantageusement dans la famille des diacides à chaîne longue.

Exemple 15 : Gourde de cycliste.

On élabore une gourde à double paroi, contenant un ALCAL à $\delta \leq 4$°C fonctionnant à une température de l'ordre de +15°C (voire plus basse selon les nécessités) ; le cycliste disposera ainsi d'une boisson fraîche pendant plusieurs heures, ce qui présente un grand avantage par rapport aux gourdes classiques.

Exemple 16 : Etui pour vaccins

On prépare un étui à double paroi dans lequel on introduit un MCPAM de température T inférieure à +15°C environ avec $\delta \leq 4$. Le MCPAM est solidifié en le plaçant dans le réfrigérateur. Cet étui peut être utilisé pour transporter un vaccin.

On mesurera l'intérêt de cette utilisation par exemple pour le randonneur qui sera muni d'un vaccin par exemple de vipère et pourra effectuer sa randonnée avec une meilleure sécurité.

L'invention fournit ainsi les moyens d'élaborer des MCF répondant à un large domaine de températures requises pour les applications industrielles.

En fonction du niveau de température auquel doit s'effectuer le changement de phase, l'homme de l'art dispose des moyens de choisir la teneur des composés organiques utilisés pour élaborer l'alliage en se conformant aux exigences définies plus haut.

## Revendications

1. Procédé d'élaboration de matériaux à changement de phases pour le maintien d'un article au cours de son utilisation à un niveau de température strict, tel que requis pour une application donnée, caractérisé par la mise en oeuvre d'un ou plusieurs alliages moléculaires, chaque alliage étant formé d'une seule phase constituant, à l'état solide, une solution solide de substitution telle que caractérisée par diffraction X, et répondant strictement à la formule (I) :

$$A_{x_a} Z_{x_z} \qquad (I)$$

dans laquelle

- A et Z sont différents et représentent des composés organiques acycliques ayant de 2 à 120 atomes de carbone, saturés ou insaturés, le cas échéant substitués, présentant la chaleur latente requise de manière classique pour être des matériaux à changement de phase,
  lesdits composés organiques A et Z ayant un degré d'homéomorphisme moléculaire $\varepsilon_K$ supérieur à 0,8 et, de préférence, supérieur à 0,90 pour les alliages binaires, ou ayant cette propriété pour les divers constituants pris deux à deux, s'il s'agit d'alliages à multicomposants,
- $x_a$ et $x_z$ représentent les proportions molaires respectivement de A et de Z, ces proportions étant ajustées pour que les composés, pour chaque alliage moléculaire, soient totalement miscibles avant la transition de phase et que dans le cas de présence de paliers d'invariance, les proportions sont ajustées pour que les composés aient des concentrations situées hors des paliers d'invariance, pour que cette transition s'effectue sur un intervalle de température précis $\delta$ n'excédant pas +6°C.

**2.** Procédé selon la revendication 1, caractérisé en ce que δ ne dépasse pas +4°C environ, et avantageusement +2°C, voire +1°C.

**3.** Procédé selon la revendication 1 ou 2, caractérisé en ce que le composé acyclique est un alcane, un alcène ou un alcyne.

**4.** Procédé selon l'une quelconque des revendications 1 à 3, caractérisé en ce que A et/ou Z comportent un ou plusieurs substituants choisis parmi les halogènes F, C1, Br, I, les groupes -OH ou $-OR_1$, les groupes alkyle, alkylène saturés ou insaturés, et alcynes, ces différents groupes ayant de préférence 1 à 8 atomes de carbone, plus spécialement de 1 à 4 atomes de carbone, les groupes -COOH, $-COOR_1$, $-COR_1$, $-CH_2OH$, $-CH(R_1)-OH$, $-C(R_1, R_2)OH$, ou leurs éthers, -CHO, -C=O, $-CONH_2$, $-NH_2$, $-NH(R_1)$, ou $-N(R_1, R_2)$, -SH, = S, $-NO_2$, $R_1$ et $R_2$, identiques ou différents l'un de l'autre, étant un groupe alkyle de 1 à 8 atomes de carbone, de préférence de 1 à 4 atomes de carbone, des groupes fonctionnels tels que amine, cétone, sulfhydryle, amide, pouvant constituer un ou plusieurs maillons de la chaîne, ces substituants occupant dans les composés organiques acycliques une position quelconque sur la chaîne carbonée et/ou, se trouvant à une extrémité de la chaîne ou aux deux extrémités.

**5.** Procédé selon la revendication 3 ou 4, caractérisé en ce que A et Z sont formés de chaînes d'alcanes le cas échéant substituées, de 8 à 100 atomes de carbone, plus spécialement de 8 à 50 atomes de carbone, consécutives ou différant de plusieurs chaînons, notamment de plus de 4 ou 5 chaînons carbonés.

**6.** Procédé selon la revendication 5, caractérisé en ce que les chaînes d'alcanes renferment un nombre pair d'atomes de carbone (Cnp).

**7.** Procédé selon la revendication 5, caractérisé en ce que A et Z sont formés de chaînes d'alcanes renfermant un nombre impair d'atomes de carbone (Cni).

**8.** Procédé selon la revendication 5, caractérisé en ce que A et Z sont formés de chaînes d'alcanes renfermant un nombre pair d'atomes de carbone et de chaînes d'alcanes renfermant un nombre impair d'atomes de carbone (Cnp et Cni).

**9.** Procédé selon la revendication 8, caractérisé en ce que une ou plusieurs chaînes d'alcanes sont substituées avec un ou plusieurs substituants tels que définis dans la revendication 4, tel qu'un groupe carboxyle ou ester ou un atome d'halogène, ce ou ces substituants occupant avantageusement l'une ou chacune des extrémités de la chaîne.

**10.** Procédé selon l'une quelconque des revendications précédentes, caractérisé en ce que lesdits alliages répondent à l'une des formules suivantes $A_{xa}\, B_{xb}$ (II), $A_{xa}\, B_{xb}\, C_{xc}$ (III), $A_{xa}\, B_{xb}\, C_{xc}\, D_{xd}$ (IV), dans lesquelles A, B, C et D, répondent aux définitions données pour la formule I, et $x_a$ à $x_d$ correspondent respectivement aux proportions molaires des différents constituants, avec $x_a + x_b = 1$ dans la formule (II), $x_a + x_b + x_c = 1$ dans la formule (III), et $x_a + x_b + x_c + x_d = 1$ dans la formule (IV).

**11.** Application des matériaux tels qu'obtenus selon l'une des revendications précédentes dans le domaine agro-alimentaire plus spécialement, pour la protection thermique et/ou le transport de denrées alimentaires typiquement de -50°C à +100°C.

**12.** Application selon la revendication 11 dans le domaine agro-alimentaire pour le transport ou la conservation de produits congelés de -50°C à -10°C environ.

**13.** Application selon la revendication 11, dans le domaine agro-alimentaire de -10°C à +6°C environ.

**14.** Application selon la revendication 11, dans le domaine agro-alimentaire de +6°C et +16°C environ.

**15.** Application selon la revendication 11, dans le domaine agro-alimentaire à des températures supérieures à +16°C, notamment jusqu'à +35°C et de +35°C à +100°C environ.

**16.** Application des matériaux tels qu'obtenus selon l'une des revendications 1 à 10, pour le conditionnement, les manipulations isothermes ou à températures contrôlées, les insuffisances fonctionnelles et thérapies symptomatiques, typiquement de -80°C à +75°C.

17. Application des matériaux tels qu'obtenus selon l'une quelconque des revendications 1 à 10, pour la protection aux fins de sécurité ou d'économie d'énergie dans une gamme de température de -80°C à +200°C et plus et pour assurer une autonomie d'emploi des ustensiles ménagers.

**Patentansprüche**

1. Verfahren zur Herstellung von Phasenänderungsmaterialien zum Halten eines Gegenstands während dessen Verwendung bei einer genauen Temperatur, wie für eine vorgegebene Anwendung erforderlich, **gekennzeichnet durch** die Verwendung einer oder mehrerer molekularer Legierungen, wobei jede Legierung aus einer Einzelphase gebildet ist, die im festen Zustand eine feste Substitutionslösung, wie durch Beugung X gekennzeichnet, darstellt und strikt der Formel (I) entspricht:

$$A_{x_a} Z_{x_z} \qquad (I)$$

worin

- A und Z verschieden sind und gesättigte oder ungesättigte, gegebenenfalls substituierte azyklische organische Verbindungen mit 2 bis 120 Kohlenstoffatomen darstellen, welche die herkömmlich erforderliche latente Wärme aufweisen, um Phasenänderungsmaterialien zu sein,

- die organischen Verbindungen A und Z einen molekularen Homöomorphiegrad $\varepsilon_K$ von mehr als 0,8 und bevorzugt mehr als 0,90 für die binären Legierungen aufweisen, oder, wenn es sich um Legierungen mit mehreren Bestandteilen handelt, diese Eigenschaft für die verschiedenen Bestandteile jeweils paarweise aufweisen,

- $x_a$ und $x_z$ die molaren Anteile von A bzw. Z darstellen, wobei diese Anteile derart eingestellt werden, daß für jede molekulare Legierung die Verbindungen vor dem Phasenübergang vollständig mischbar sind, und für den Fall des Vorhandenseins von Invarianzbereichen die Anteile derart eingestellt werden, daß die Verbindungen Konzentrationen außerhalb der Invarianzbereiche aufweisen, so daß dieser Übergang in einem exakten Temperaturbereich $\delta$, der +6°C nicht übersteigt, verläuft.

2. Verfahren nach Anspruch 1, **dadurch gekennzeichnet, daß** $\delta$ etwa +4°C und bevorzugt +2°C , sogar +1°C nicht übersteigt.

3. Verfahren nach Anspruch 1 oder 2, **dadurch gekennzeichnet, daß** die azyklische Verbindung ein Alkan, ein Alken oder ein Alkin ist.

4. Verfahren nach einem der Ansprüche 1 bis 3, **dadurch gekennzeichnet, daß** A und/oder Z einen oder mehrere Substituenten aufweisen, ausgewählt aus den Halogenen F, Cl, Br, I, den Gruppen -OH oder -$OR_1$, den gesättigten oder ungesättigten Alkyl- und Alkylengruppen und den Alkingruppen, wobei diese verschiedenen Gruppen bevorzugt 1 bis 8, stärker bevorzugt 1 bis 4 Kohlenstoffatome aufweisen, den Gruppen -COOH, -$COOR_1$, -$COR_1$, -$CH_2OH$, -$CH(R_1)$-OH, -$C(R_1, R_2)OH$, oder deren Ethern, -CHO, -C=O, -$CONH_2$, -$NH_2$, -$NH(R_1)$ oder -$N(R_1, R_2)$, -SH, =S, -$NO_2$, wobei $R_1$ und $R_2$ gleich oder voneinander verschieden sind und eine Alkylgruppe von 1 bis 8 Kohlenstoffatomen, bevorzugt 1 bis 4 Kohlenstoffatomen sind, wobei funktionelle Gruppen wie etwa Amin, Keton, Sulfhydryl, Amid ein oder mehrere Glieder in der Kette darstellen können, und wobei diese Substituenten in den azyklischen organischen Verbindungen eine beliebige Position auf der Kohlenstoffkette einnehmen können und/ oder sich an einem Ende der Kette oder den zwei Enden befinden können.

5. Verfahren nach Anspruch 3 oder 4, **dadurch gekennzeichnet, daß** A und Z aus gegebenenfalls substituierten Alkanketten von 8 bis 100 Kohlenstoffatomen, insbesondere 8 bis 50 Kohlenstoffatomen, aufeinanderfolgend oder andersartig, aus mehreren Ketten, insbesondere mehr als vier oder fünf Kohlenstoffketten, gebildet sind.

6. Verfahren nach Anspruch 5, **dadurch gekennzeichnet, daß** die Alkanketten eine geradzahlige Anzahl von Kohlenstoffatomen umfassen (Cnp).

7. Verfahren nach Anspruch 5, **dadurch gekennzeichnet, daß** A und Z aus Alkanketten, die eine ungeradzahlige

Anzahl von Kohlenstoffatomen enthalten (Cni), gebildet werden.

8. Verfahren nach Anspruch 5, **dadurch gekennzeichnet, daß** A und Z aus Alkanketten, die eine geradzahlige Anzahl von Kohlenstoffatomen umfassen, und aus Alkanketten, die eine ungeradzahlige Anzahl von Kohlenstoffatomen umfassen(Cnp und Cni), gebildet werden.

9. Verfahren nach Anspruch 8, **dadurch gekennzeichnet, daß** eine oder mehrere Alkanketten mit einem oder mehreren Substituenten wie in Anspruch 4 definiert, wie etwa einer Carboxyl- oder Ethergruppe oder einem Halogenatom substituiert sind, wobei der oder die Substituenten vorzugsweise eines oder beide Enden der Kette einnehmen.

10. Verfahren nach einem der vorhergehenden Ansprüche, **dadurch gekennzeichnet, daß** die Legierungen einer der nachfolgenden Formeln $A_{xa}\ B_{xb}$ (II), $A_{xa}\ B_{xb}\ C_{xc}$ (III), $A_{xa}\ B_{xb}\ C_{xc}\ D_{xd}$ (IV) entsprechen, in denen A, B, C und D den in Formel (I) angegebenen Definitionen entsprechen und $x_a$ bis $x_d$ jeweils den molaren Anteilen der verschiedenen Bestandteile entsprechen, wobei $x_a + x_b = 1$ in Formel (II), $x_a + x_b + x_c = 1$ in Formel (III), und $x_a + x_b + x_c + x_d = 1$ in Formel (IV).

11. Verwendung von Stoffen wie nach einem der vorhergehenden Ansprüche erhalten auf dem Agrarnahrungsmittel-Gebiet, insbesondere zum thermischen Schutz und/oder Transport von Lebensmitteln bei typischerweise -50°C bis 100°C.

12. Verwendung nach Anspruch 11 auf dem Agrarnahrungsmittel-Gebiet zum Transport oder zur Konservierung gefrorener Produkte bei etwa -50°C bis -10°C.

13. Verwendung nach Anspruch 11 auf dem Agrarnahrungsmittel-Gebiet bei etwa -10°C bis +6°C.

14. Verwendung nach Anspruch 11 auf dem Agrarnahrungsmittel-Gebiet bei etwa +6°C bis +16°C.

15. Verwendung nach Anspruch 11 auf dem Agrarnahrungsmittel-Gebiet bei Temperaturen von mehr als +16°C, insbesondere bis zu 35°C und von etwa 35°C bis zu 100°C.

16. Verwendung von Stoffen wie nach einem der Ansprüche 1 bis 10 erhalten zur der Klimatisierung, isothermen Handhabungen und Handhabungen bei kontrollierter Temperatur, funktionellen Unzulänglichkeiten und symptomatischen Therapien, typischerweise bei -80°C bis + 75°C.

17. Verwendung von Stoffen wie nach einem der Ansprüche 1 bis 10 erhalten zum Schutz bei Sicherheits- oder Energiesparzwecken in einem Temperaturbereich von -80°C bis 200°C und mehr, und um eine unabhängige Verwendung von Haushaltsgeräten zu gewährleisten.

## Claims

1. A method for making phase change materials for maintaining an article, during its use, at a strict temperature level, such as required for a given application, comprising using a composition formed by one or more molecular alloys, each alloy being formed of a single phase which constitutes, at the solid state, a solid solution of substitution such as characterized by X diffraction, and strictly having formula (I)

$$A_{x_a}\ Z_{x_z} \qquad (I)$$

wherein

- A and Z are different and represent, saturated or unsaturated, optionally substituted, acyclic organic compounds, having from 2 to 120 carbon atoms, having the latent heat conventionally required to be a phase change material,
  said organic compounds A and Z having a degree of molecular homeomorphism $\varepsilon_k$ higher than 0.8 and, preferably, higher than 0.90 for binary alloys, or having said property for the various constituents taken 2 by 2,

when they are multi-component alloys,

- $x_a$ and $x_z$ represent the molar proportions of A and Z respectively

said proportions being adjusted so that the compounds, for each molecular alloy, be totally miscible prior to the phase transition and that in the case of invariance levels, the proportions be adjusted so that the compounds have concentrations outside the invariance levels, so that the transition occurs on a precise temperature range δ not exceeding +6°C.

**2.** The method of claim 1, wherein 6 does not exceed about + 4°C, and advantageously +2°C, even +1°C.

**3.** The method of claim 1 or 2, wherein the acyclic compound is an alkane, an alkene or an alkyne.

**4.** The method according to anyone of claims 1 to 3, wherein A and/or Z contain one or more substituents selected from the halogens F, Cl, Br, I, the -OH or -$OR_1$ groups, the alkyl, saturated or unsaturated alkylene, and alkyne groups, these various groups preferably having 1 to 8 carbon atoms, more especially from 1 to 4 carbon atoms, the -COOH, -$COOR_1$, -$COR_1$, -$CH_2OH$, -$CH(R_1)$ -OH, -$C(R_1, R_2)OH$, or the ethers thereof, -CHO, -C=O, -$CONH_2$, -$NH_2$, -$NH(R_1)$, or -$N(R_1,R_2)$, -SH, = S, -$NO_2$ groups, $R_1$ and $R_2$, which are identical or different, being an alkyl group having 1 to 8 carbon atoms, preferably from 1 to 4 carbon atoms, functional groups such as amine, ketone, sulfhydryl, and amide, which can constitute one or several links of the chain, said substituents occupying in the acyclic organic compounds any position on the carbon chain and/or being at one end of the chain or at both ends.

**5.** The method according to claim 3 or 4, wherein A and Z are formed of optionally substituted chains of alkanes, from 8 to 100 carbon atoms, more especially from 8 to 50 carbon atoms, consecutive or differing from several links, particularly of more than 4 or 5 carbon links.

**6.** The method of claim 5, wherein the chains of alkane comprise an odd number of carbon atoms (Cnp).

**7.** The method of claim 5, wherein A and Z are formed of chains of alkane comprising an even number of carbon atoms (Cni).

**8.** The method of claim 5, wherein A and Z are formed of chains of alkanes containing an odd number of carbon atoms and of chains of alkanes containing an even number of carbon atoms (Cnp and Cni).

**9.** The method of claim 8, wherein one or several chains of alkanes are substituted by one or several substituents such as defined in claim 4, such as a carboxy or ester group or an halogen atom, said substituent(s) advantageously occupying one or each end of the chain.

**10.** The method according to anyone of the preceeding claims, wherein said alloys have one of the following formulae $A_{xa}\ B_{xb}$ (II), $A_{xa}\ B_{xb}\ C_{xc}$ (III), $A_{xa}\ B_{xb}\ C_{xc}\ D_{xd}$ (IV), in which A, B, C and D correspond to the definitions given for formula I, and $x_a$ to $x_d$ correspond to the molar proportions of the various constituents respectively, with $x_a + x_b = 1$ in formula (II), $x_a + x_b + x_c = 1$ in formula (III) and $x_a + x_b + x_c + x_d = 1$ in formula (IV).

**11.** The use of the materials such as obtained according to anyone of the preceeding claims, more especially in the field of agricultural feedstuffs for thermal protection and/or transport of feedstuffs, typically from -50°C to +100°C.

**12.** Use according to claim 11 in the agricultural feedstuffs field for the transport or preservation of frozen products from about -50°C to -10°C.

**13.** Use according to claim 11 in the agricultural feedstuffs field from about -10°C to +6°C.

**14.** Use according to claim 11 in the agricultural feedstuffs field between about +6°C to +16°C.

**15.** Use according to claim 11 in the agricultural feedstuffs field at temperatures higher than + 16°C, particularly up to 35°C and from about +35°C to +100°C.

**16.** Application of the materials according to claim 11, for packaging, isothermal or controlled temperature handling, functional difficiencies and symptomatic therapies, from -80°C to +75°C.

**17.** Use of the materials according to anyone of claims 1 to 10, for protection for safety or energy saving aims over a temperature range from -80°C to +200°C, and to allow autonomy of use of domestic articles.

FIGURE 1

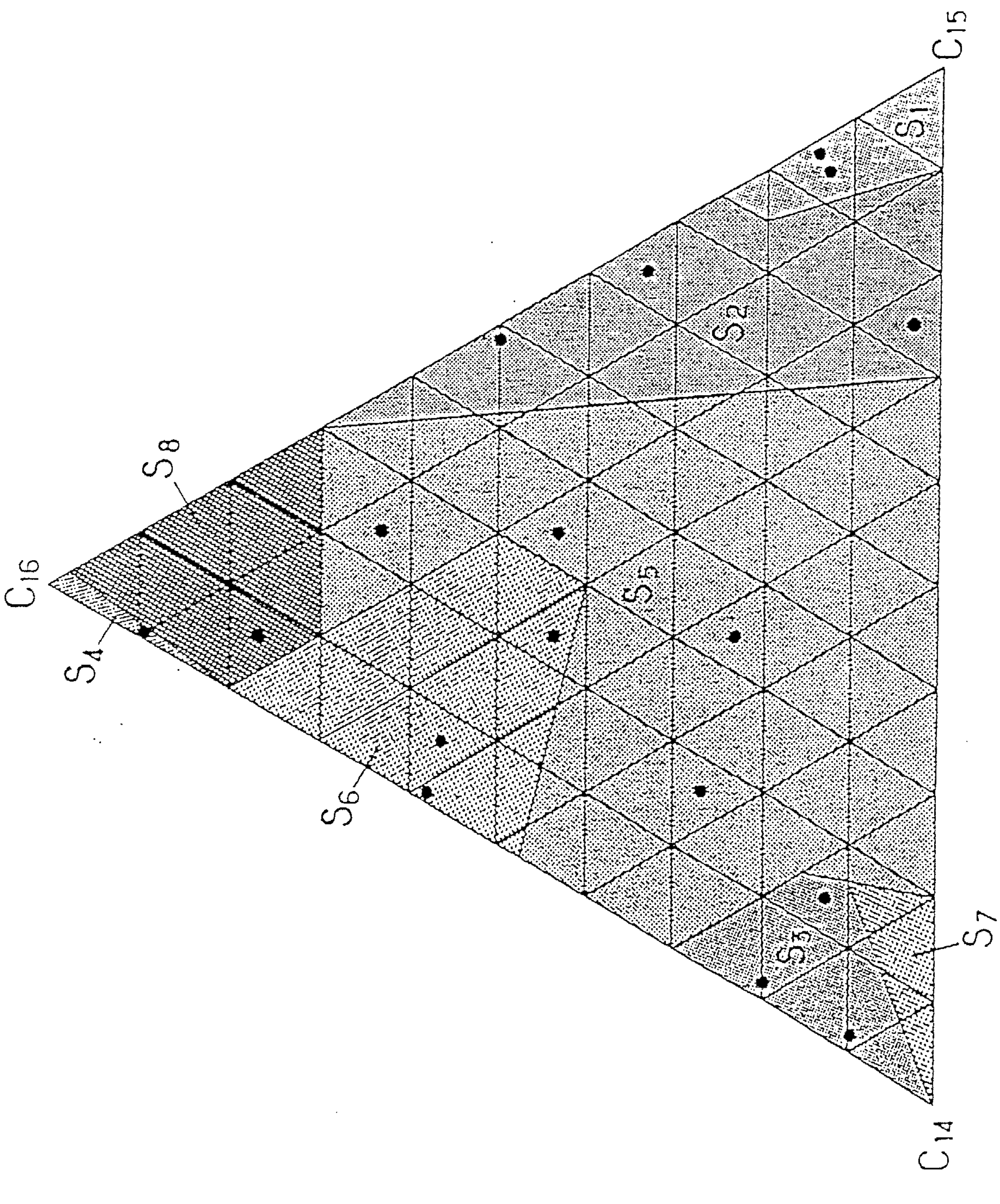

FIGURE 2

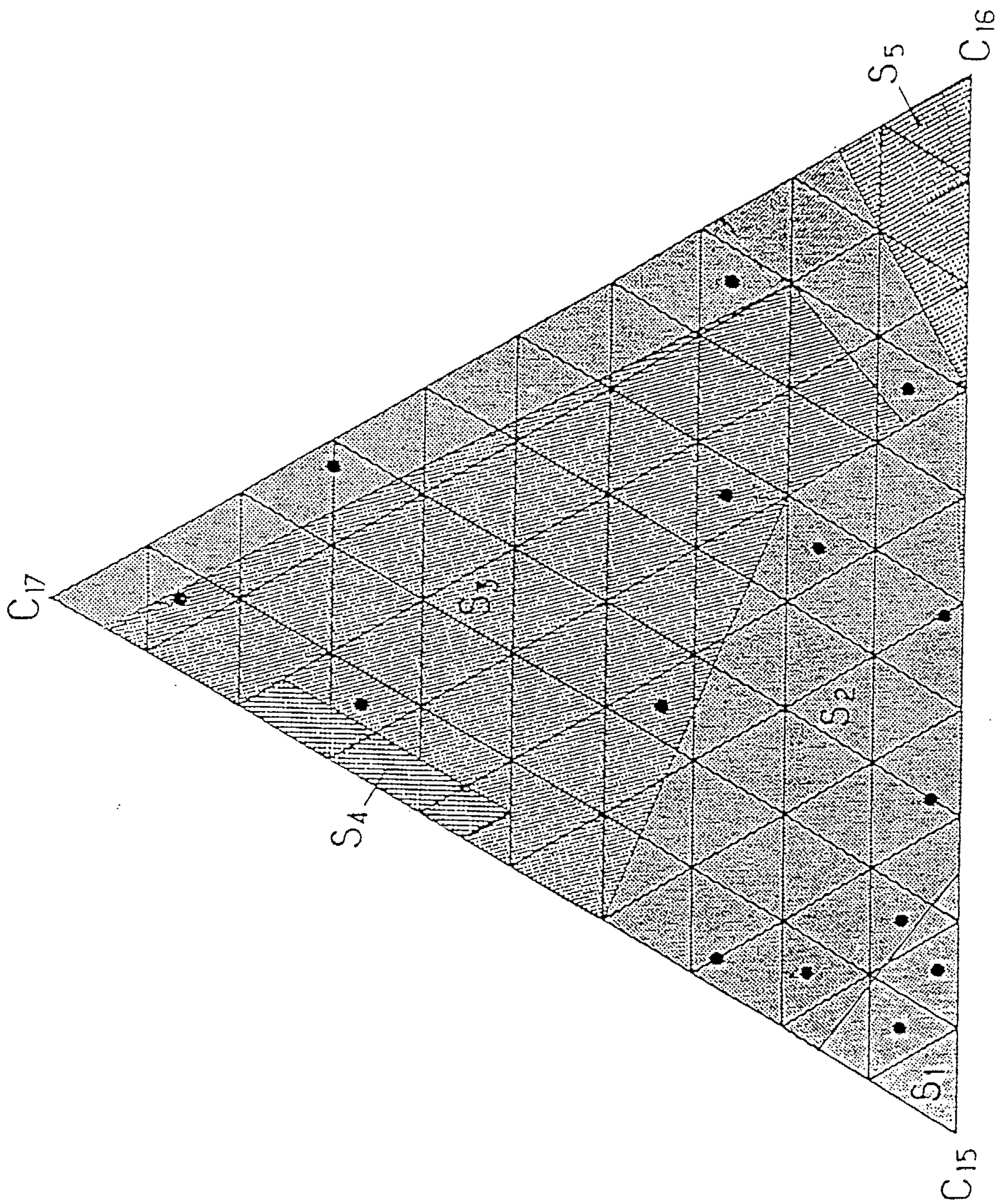

FIGURE 3

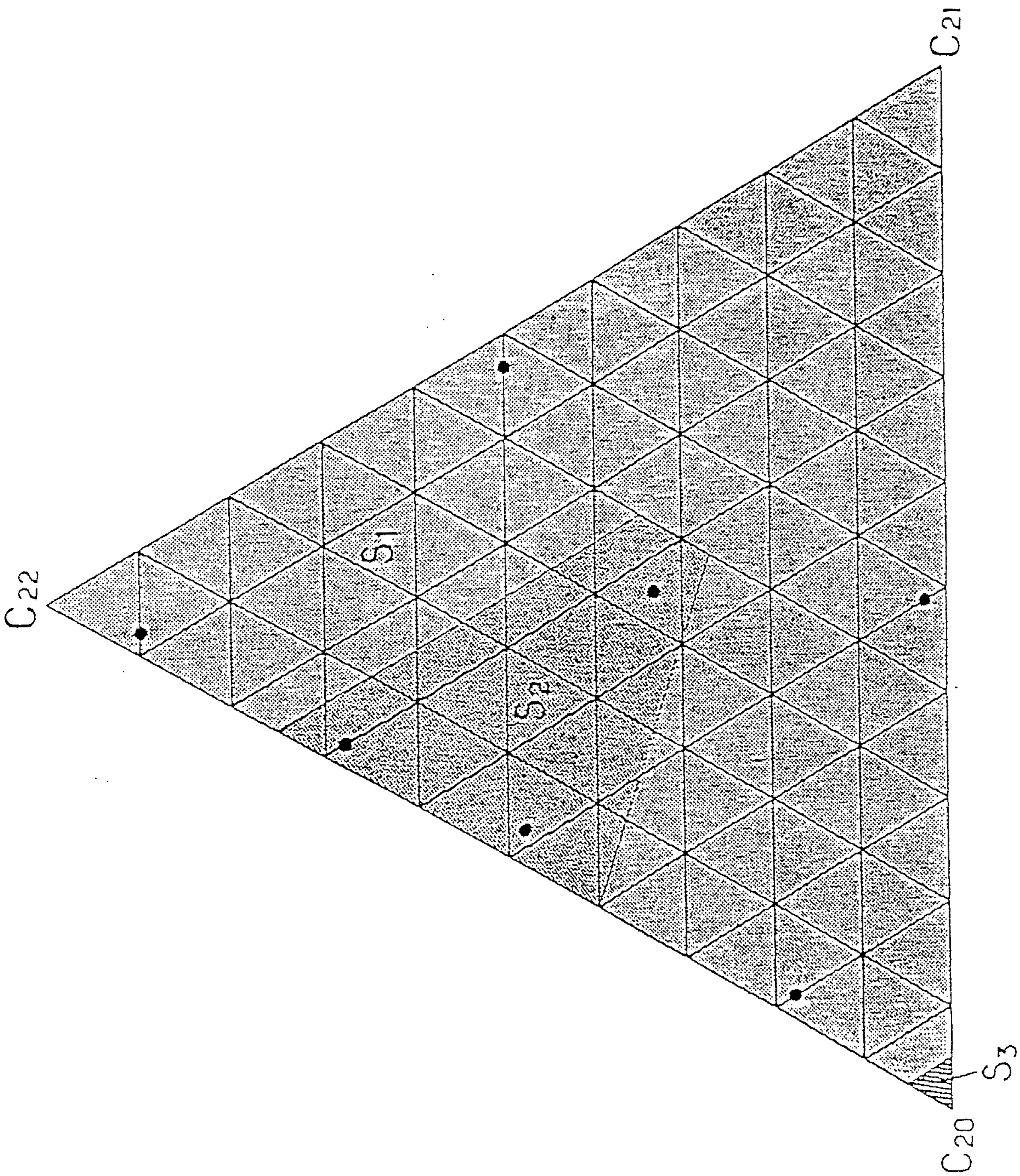

FIGURE 4

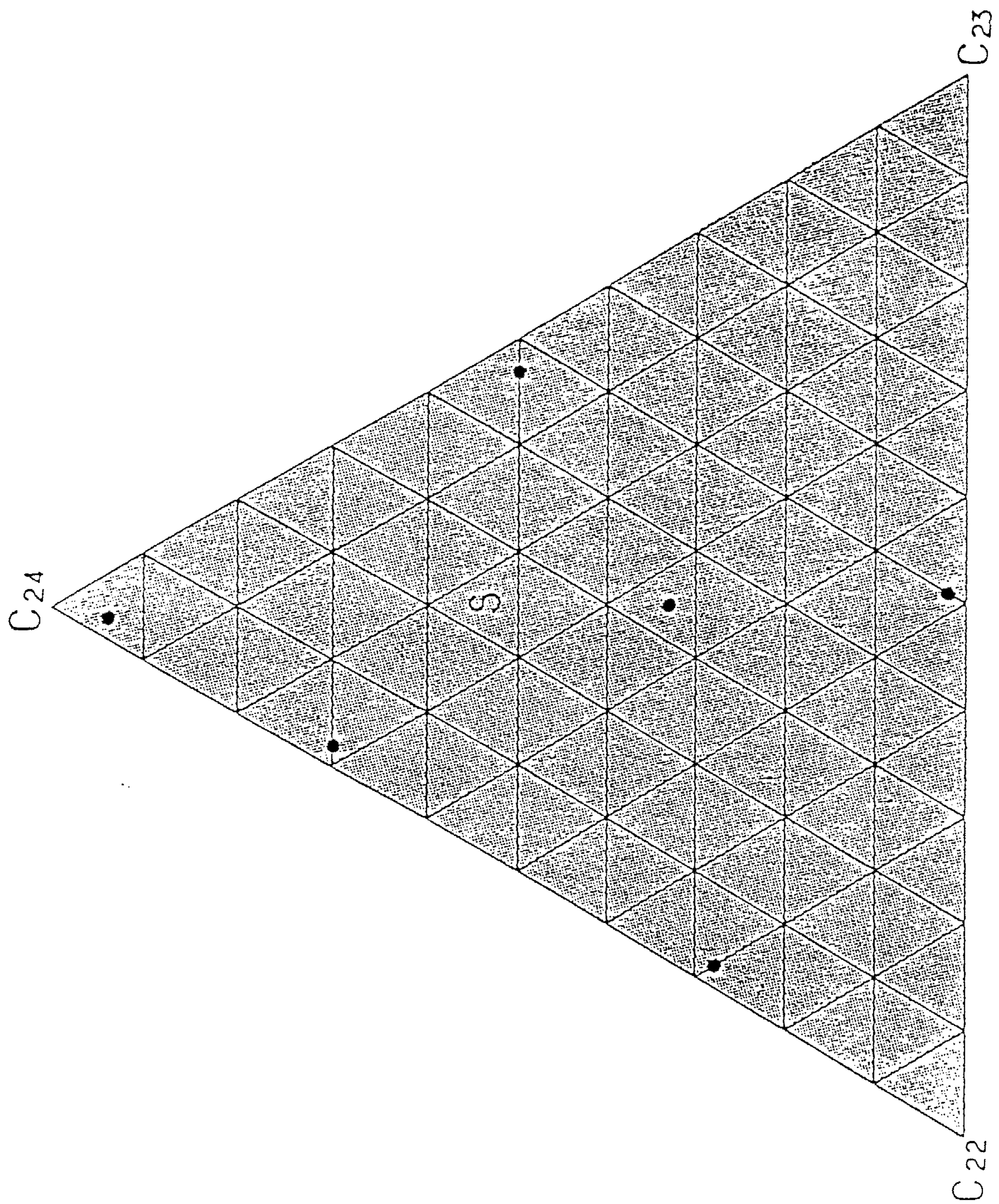

FIGURE 5

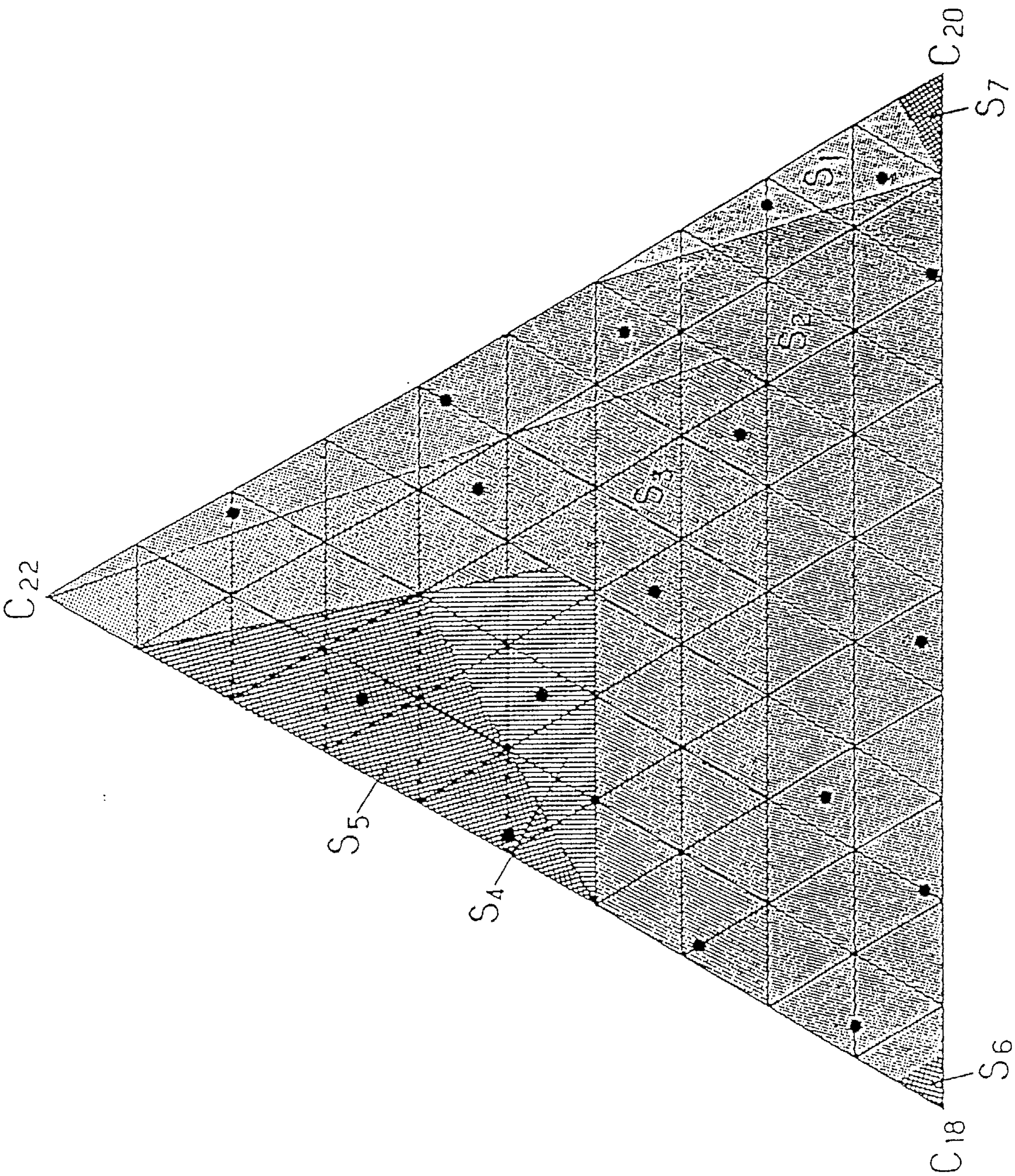

FIGURE 6

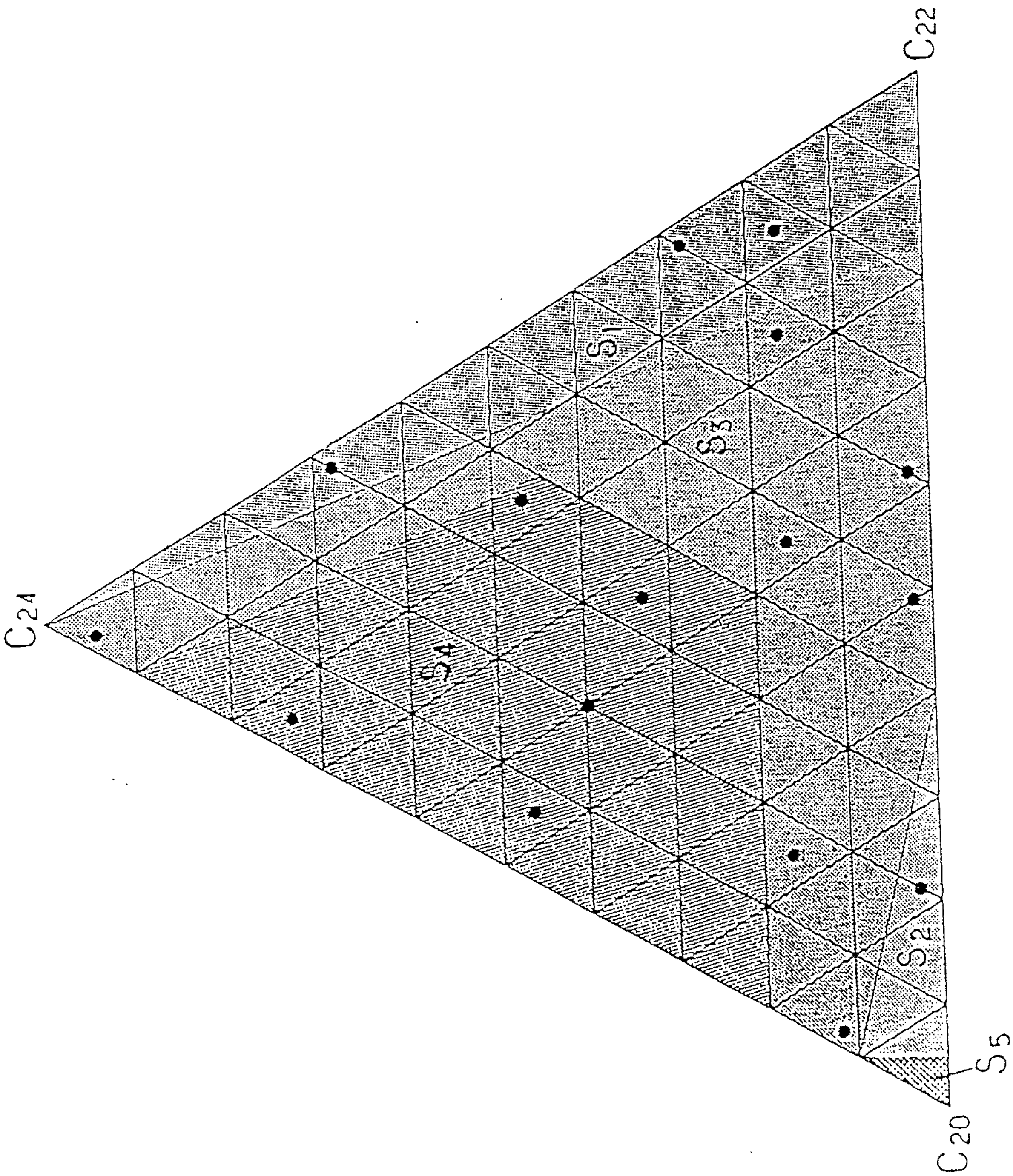

FIGURE 7

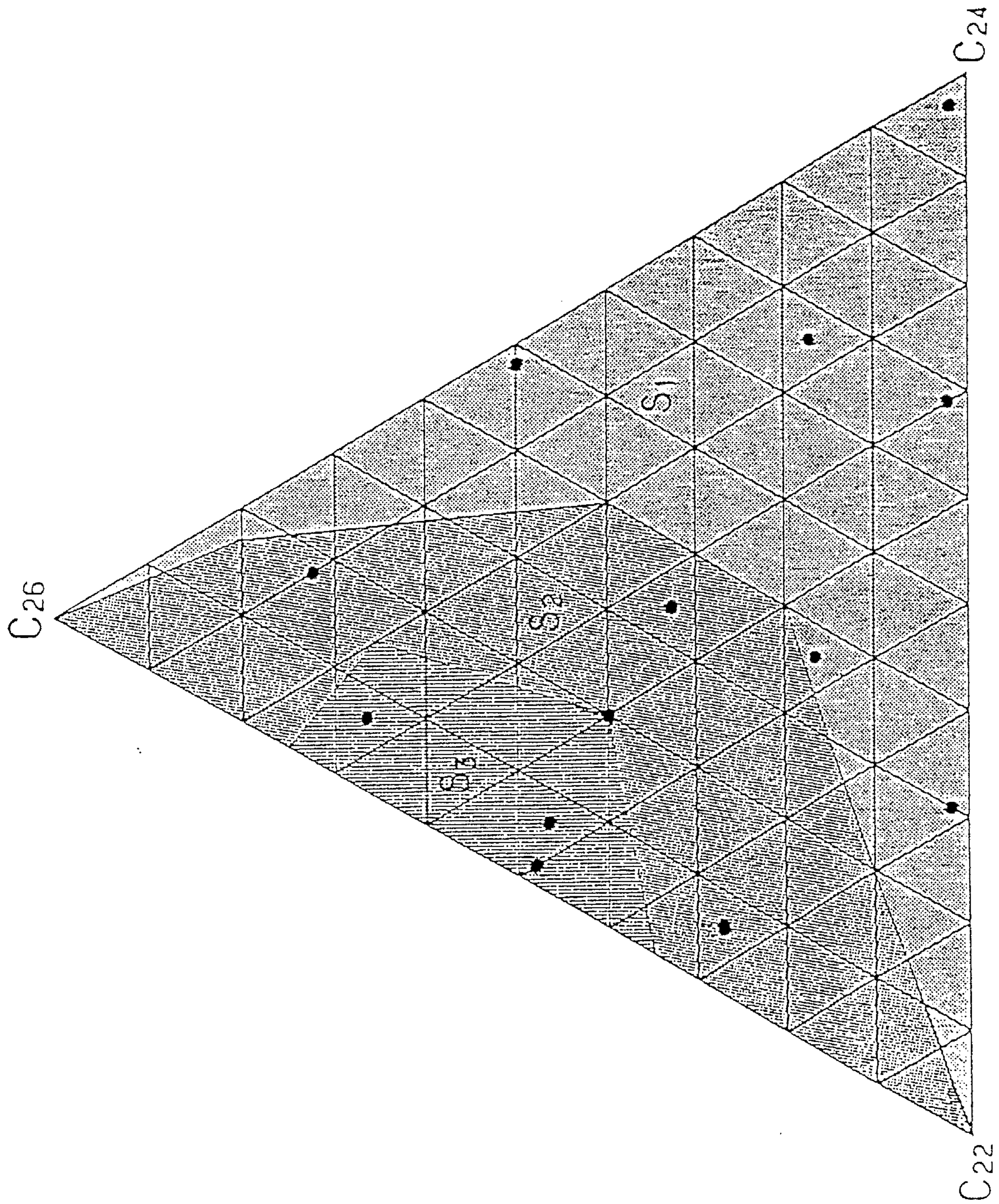

FIGURE 8

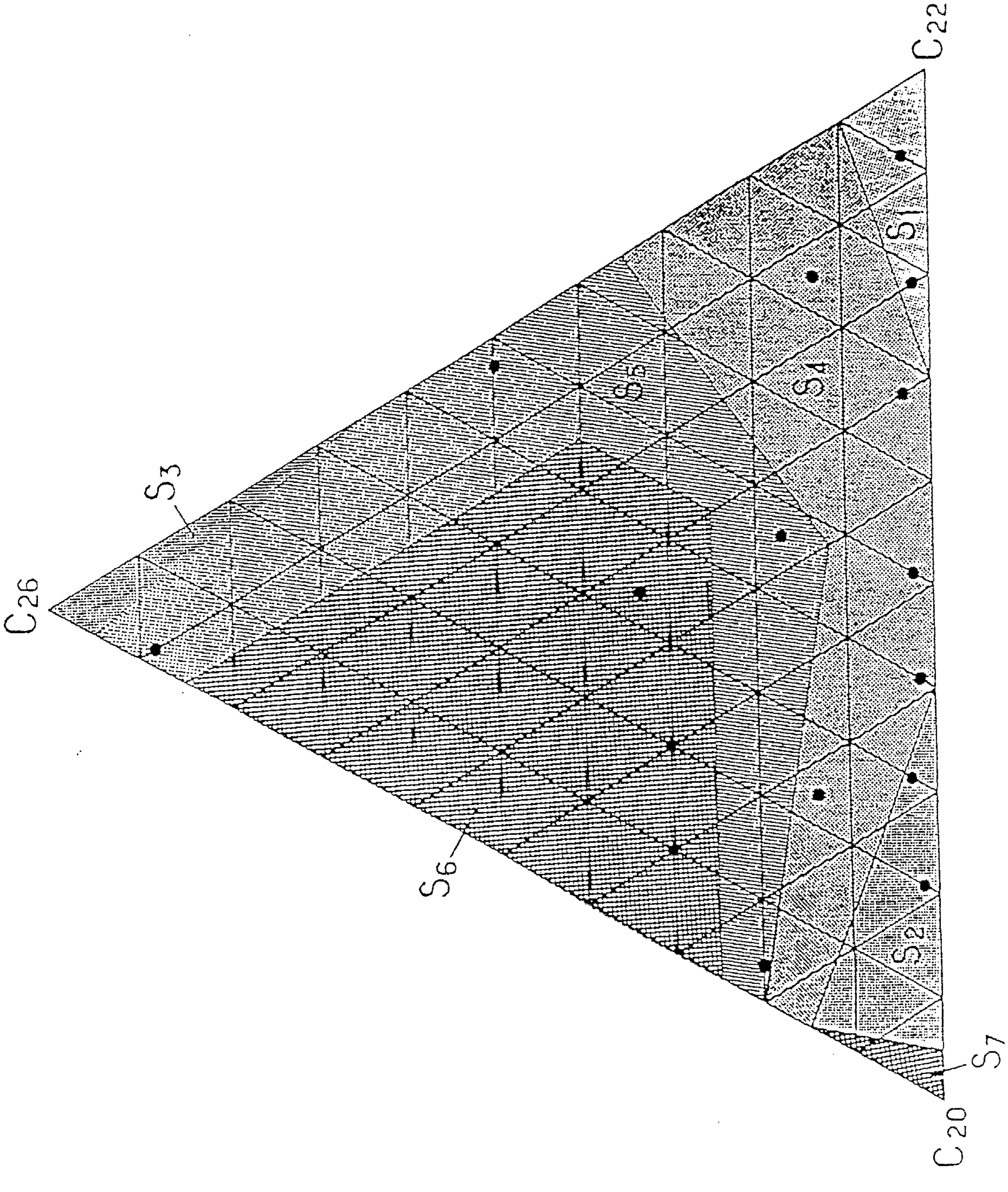

FIGURE 9